# EUROPEAN PATENT SPECIFICATION

(11) **EP 4 347 576 B1**
(45) Date of publication and mention of the grant of the patent: **23.04.2025**
(21) Application number: 22732100.7
(22) Date of filing: 02.06.2022
(51) Int. Cl.: C07D 295/08, A61K 31/4155, A61P 25/28, C07D 261/08, C07D 413/06

(54) **COMPOUNDS AND PHARMACEUTICAL COMPOSITIONS FOR USE IN NEURODEGENERATIVE DISORDERS**
VERBINDUNGEN UND PHARMAZEUTISCHE ZUSAMMENSETZUNGEN ZUR VERWENDUNG IN NEURODEGENERATIVEN ERKRANKUNGEN
COMPOSÉS ET COMPOSITIONS PHARMACEUTIQUES À UTILISER POUR DES TROUBLES NEURODÉGÉNÉRATIFS

(30) Priority: 02.06.2021 EP 21177320
(43) Date of publication of application: 10.04.2024
(73) Proprietor: Universiteit Hasselt, 3500 Hasselt (BE); Universiteit Maastricht, 6211 LK Maastricht (NL); Academisch ziekenhuis Maastricht, 6229 HX Maastricht (NL)
(72) Inventor: VANMIERLO, Tim, 3500 Hasselt (BE); SCHEPERS, Melissa, 3500 Hasselt (BE); PICCART, Elisabeth, 3500 Hasselt (BE); PRICKAERTS, Jos, 6229 ER Maastricht (NL)
(74) Representative: Arnold & Siedsma
(86) International application number: PCT/EP2022/065055
(87) International publication number: WO 2022/253959

(56) References cited:
- EP-A1- 2 907 806
- CHIARA BRULLO ET AL: "New insights into selective PDE4D inhibitors: 3-(Cyclopentyloxy)-4-methoxybenzaldehyde O-(2-(2,6-dimethylmorpholino)-2-oxoethyl) oxime (GEBR-7b) structural development and promising activities to restore memory impairment", EUROPEAN JOURNAL OF MEDICINAL CHEMISTRY, vol. 124, 1 November 2016 (2016-11-01), AMSTERDAM, NL, pages 82 - 102, XP055511566, ISSN: 0223-5234, DOI: 10.1016/j.ejmech.2016.08.018

## Description

### FIELD OF THE INVENTION

The present invention relates to compounds, pharmaceutical compositions and medicaments suitable for use in the prevention and/or treatment of neurodegenerative disorders, such as for example demyelinating diseases. These compounds improve cognition and/or neuroplasticity as well as to induce and/or stimulate remyelination of the myelin sheet. Furthermore, the present invention relates to methods for synthesis of said compounds.

### BACKGROUND TO THE INVENTION

Neurodegenerative diseases are associated with symptoms when neurons degenerate, lose function or die. Since these are mainly progressive, the consequences of neurodegenerative diseases are very devastating. Patients with neurodegenerative diseases suffer from severe degeneration in cognitive or motor skills. Neurodegeneration can be found in the brain at many levels of neuronal circuitry, ranging from molecular to systemic neurodegeneration. Because there is presently no known way to reverse the progressive degeneration of neurons, these diseases are considered to be incurable. Therefore, patients have limited quality of life or expectation of life at all.

The most commonly known neurodegenerative diseases are Alzheimer's disease (AD), Parkinson's disease (PD), Huntington's disease, Lou Gehrig's disease (ALS), stroke, traumatic brain injury, spinal cord injury, mild cognitive impairment and demyelinating diseases such as peripheral demyelinating diseases, and multiple sclerosis (MS).

Demyelinating diseases are a group of neurological/neurodegenerative disorders in which myelin, the substance surrounding axons of neurons, degenerates. As a result, the axon's ability to conduct electrical signals degenerates. The most common demyelinating disease is multiple sclerosis, a demyelinating disorder of the central nervous system (CNS). Demyelinating diseases can also be related to the peripheral nervous system (i.e. peripheral demyelinating diseases), such as for example different types of neuropathy, Marie-Charcot tooth disease or traumatic nerve injury.

Early in the course of demyelinating disorders, such as multiple sclerosis (MS), neuroinflammation not only induces demyelination but at the same time activates endogenous repair mechanisms (remyelination). Early remyelination is characterized by the expansion and mobilization of oligodendrocyte progenitor cells (OPCs). OPCs rapidly remyelinate affected axons, yielding remyelinated shadow plaques in the central nerve system (CNS). Despite the presence of sufficient numbers of OPCs in the vicinity of the pathological lesions, endogenous repair mechanisms frequently fail in pMS, resulting in chronically demyelinated axons embedded in gliotic scar tissue. This has profound pathophysiological consequences. Loss of myelin not only disrupts axonal function per se, but it also compromises the physical integrity of axons by increasing susceptibility to inflammatory mediators and disrupting trophic support provided by myelinating oligodendrocytes.

Phosphodiesterases (PDEs) are a class of enzymes that hydrolyze and inactivate cyclic oligonucleotides (cAMP and/or cGMP). Cyclic oligonucleotides are second messengers that translate an extracellular signal such as a growth factor binding to its receptor into cellular differentiation. PDEs have been classified in 11 families (PDE1-11) based on subcellular distribution, mechanisms of regulation, and enzymatic and kinetic properties. Most of these families consist of several gene products (e.g. PDE4A-4D), yielding a cell type-specific PDE expression signature. Previously, it has been shown that aspecific inhibition of PDE4 supports OPC differentiation and neuronal survival in a model of spinal cord injury, reduces neuroinflammation in an animal model for MS, and improves neuroplasticity and cognitive parameters such as learning and memory in different species.

The genes PDE4A and PDE4B are known to show a higher expression level in oligodendrocytes, compared to the PDE4D gene, which shows a 10-fold lower expression. The pan-PDE4 inhibitor roflumilast, which inhibits all PDE4 isoforms, induces in vitro and in vivo remyelination as well as an improved cognitive behavior.

The present invention aims at enabling efficient and effective treatment, or preferably curing, of neurodegenerative disorders. Even though the currently available therapeutics are effective in the initial stages of neurodegenerative diseases, they have limited efficacy in preventing the transition towards the progressive stage when cognitive impairment prevails, nor can they ensure recovery of the myelin sheath. During the chronic stages, when little inflammation is present, endogenous repair mechanisms (remyelination) become insufficient and cause a gradually worsening of the disability. We have shown that inhibition of phosphodiesterase 4D boosts OPC differentiation, and therefore, myelination, without inducing side-effects. Herein we have identified compounds which stay available for a longer period of time in the brain and are therefore excellent candidates to induce OPC differentiation and remyelination. Indeed, we show that treatment of primary OPCs with RICE01 (i.e. compound of formula I') induces OPC differentiation and myelin production.

Document EP2907806 A1 discloses PDE4D inhibitors.

### SUMMARY OF THE INVENTION

Accordingly, in a first aspect, the present invention provides a compound according to formula (I), or a stereoisomer, tautomer, racemic, salt, hydrate, N-oxide form, or solvate thereof, 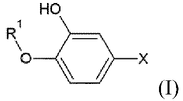 wherein:
R¹ = -CH₃ or -CHF₂;
X= 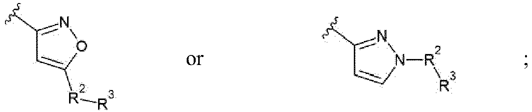
R² = -CO, -C=O(CH₂), -CH(OH)-CH₂, -CH₂-C=O, -CH₂-CH₂-C=O, -CH₂-CH(OH)-CH₂, -CH₂CH(OCOR⁴)-CH₂;
R³ = 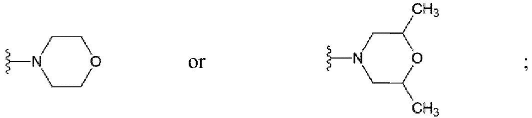 and
R⁴ = C₁-C₃ alkyl,

In a particular embodiment, the present invention provides a compound as defined herein wherein R⁴ is -CH₃.

In yet a further embodiment, the compound of the present invention is selected from the list comprising:

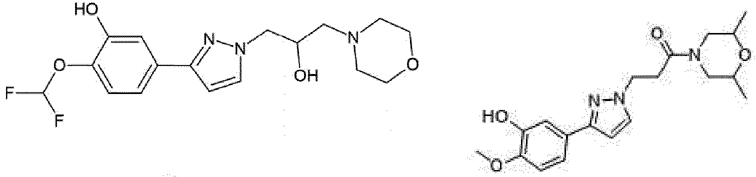

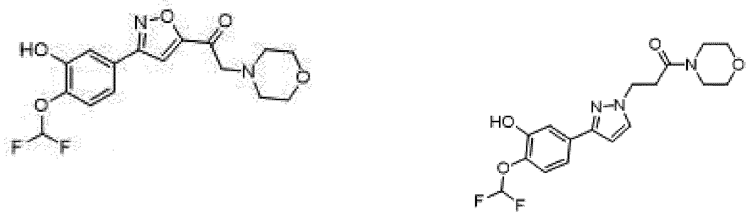

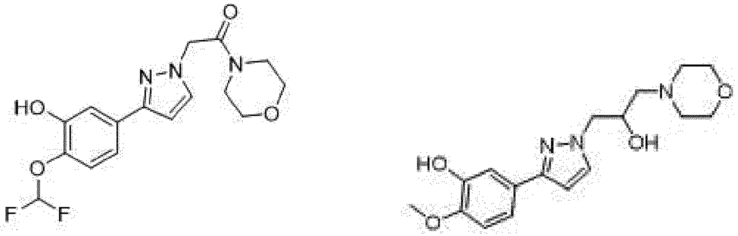

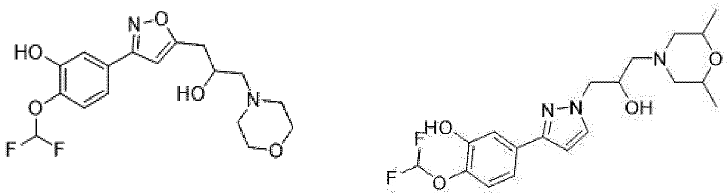

In yet a further embodiment, the compound of the present invention is represented by formula (I')

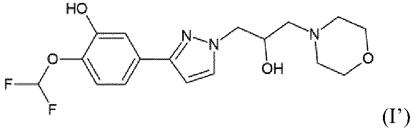

In a further aspect, the present invention provides a pharmaceutical composition comprising at least one compound as defined herein and a pharmaceutical acceptable carrier.

The present invention also provides the compound or pharmaceutical composition as defined herein for use in human or veterinary medicine; in particular for use as a medicament with PDE4D inhibiting activity.

In a further embodiment, the present invention provides a compound or pharmaceutical composition as defined herein for use in the prevention and/or treatment and/or cure of a neurodegenerative disorder in a subject; in particular a neurodegenerative disorder selected from the list comprising: Alzheimer's disease (AD), Parkinson's disease (PD), Huntington's disease, Lou Gehrig's disease (ALS), stroke, traumatic brain injury, spinal cord injury, mild cognitive impairment and demyelinating diseases such as peripheral demyelinating diseases, and multiple sclerosis (MS).

In a specific embodiment, said multiple sclerosis is progressive multiple sclerosis such as selected from the group comprising primary progressive multiple sclerosis and secondary progressive multiple sclerosis, or relapse remitting multiple sclerosis.

The present invention also provides a PDE4D inhibitor comprising a compound or a pharmaceutical composition as defined herein, wherein the PDE4D inhibitor selectively inhibits the type D isoforms of PDE4.

In a further aspect, the present invention provides a method for synthesis of a compound as defined herein, comprising the steps of:
- providing a compound according to formula (II); 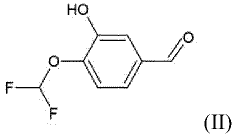
- reacting the compound according to formula (II) with 4-Methoxybenzyl chloride (PMBCl) forming a compound according to formula (III); 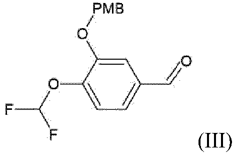
- dissolving the compound according formula (III) in acetonitrile (MeCN);
- reacting the dissolved compound according to formula (III) to form the compound according to formula (IV), 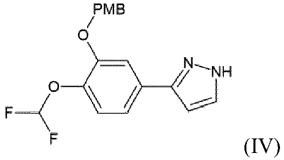
wherein the step of reacting comprises:
- adding toluene-4-sulfonic acid hydrazide to the dissolved compound according to formula (III) forming a reaction mixture;
- adding sodium hydroxide (NaOH) to the reaction mixture; and
- adding 1-vinlyimidazole to the reaction mixture;
- reacting the compound according formula (IV) with 2-(chloromethyl)oxirane and trimethylamine (Et₃N) to form the compound according to formula (V); 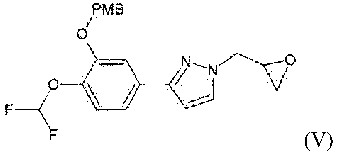
- reacting the compound according formula (V) with morpholine to form the compound according to formula (VI); and 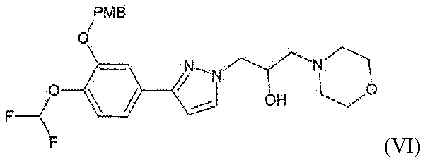
- reacting the compound according to formula (VI) with trifluoroacetic acid (TFA) to form the compound according to formula (I').

In a specific embodiment, the step of adding toluene-4-sulfonic acid hydrazide to the dissolved compound according to formula (III) forming a reaction mixture is performed at a temperature in the range of 15 °C to 40 °C, preferably at a temperature in the range of 15 °C to 30 °C, more preferably at a temperature of about 20°C.

In yet a further embodiment, the step of adding toluene-4-sulfonic acid hydrazide to the dissolved compound according to formula (III) forming a reaction mixture is performed for at least 45 minutes, more preferably performed for at least 55 minutes, more preferably performed for about 60 minutes.

In a further embodiment, the step of adding sodium hydroxide (NaOH) to the reaction mixture is performed for at least 10 minutes, preferably for at least 15 minutes, more preferably for at least 20 minutes.

In yet another embodiment, the step of reacting the compound according formula (V) with morpholine to form the compound according to formula (VI) is performed at a temperature in the range of 30 °C to 80 °C, preferably performed at a temperature in the range of 40 °C to 70 °C, more preferably performed at a temperature in the range of 50 °C to 60 °C.

### DETAILED DESCRIPTION

Accordingly, as already defined herein above, the present invention provides a compound according to formula (I), 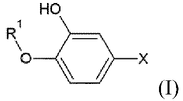 wherein:
R¹ = -CH₃ or -CHF₂; 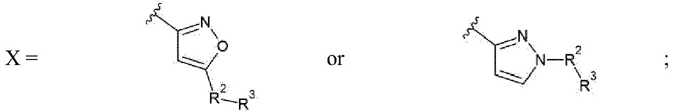
R² = -CO, -C=O(CH₂), -CH(OH)-CH₂, -CH₂-C=O, -CH₂-CH₂-C=O, -CH₂-CH(OH)-CH₂, -CH₂CH(OCOR⁴)-CH₂; 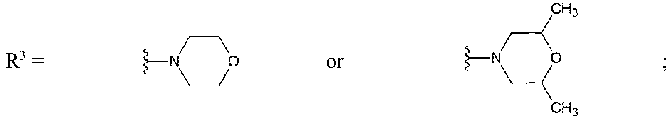 and
R⁴ = C₁-C₃ alkyl,
   or a stereoisomer, tautomer, racemic, salt, hydrate, N-oxide form, or solvate thereof.

The present invention further provides a compound according to formula (I), 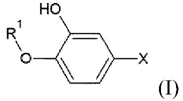 wherein:
R¹ = -CH₃ or -CHF₂;
X= 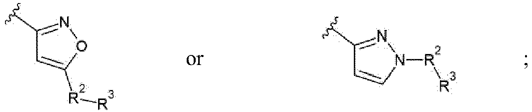
R² = -CO, -C=O(CH₂), -CH(OH)-CH₂, -CH₂-C=O, -CH₂-CH₂=O, -CH₂-CH(OH)-CH₂, -CH₂CH(OCOR⁴)-CH₂; 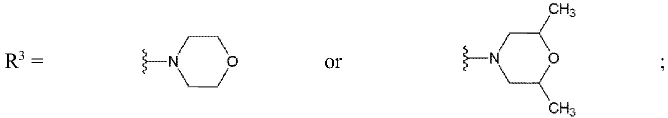 and
R⁴ = C₁-C₃ alkyl,
   or a stereoisomer, tautomer, racemic, salt, hydrate, N-oxide form, or solvate thereof.

In a preferred embodiment according to the invention, R⁴ of the compound according to formula (I) is -CH₃.

It is noted that -CH₃ refers to a methyl group.

In yet a further embodiment, the compound of the present invention is selected from the list comprising: 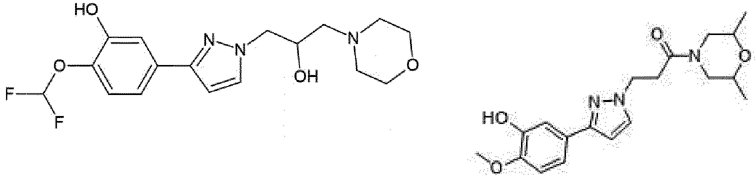 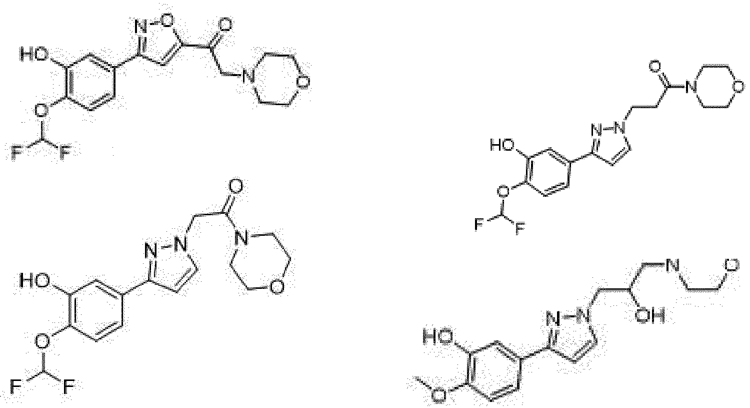 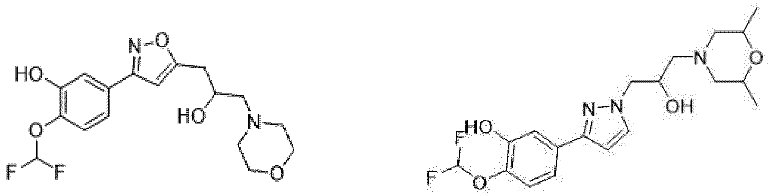 In yet another preferred embodiment according to the invention, formula (I) is

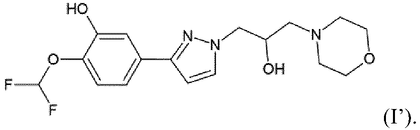

The invention also relates to Compound according to formula (I), or a stereoisomer, tautomer, racemic, salt, hydrate, N-oxide form, or solvate thereof 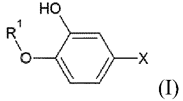 wherein:
R¹ = -CH₃ or -CHF₂;
X= 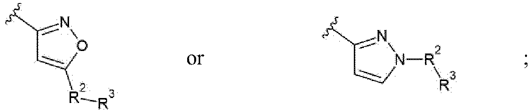
R² = -CO, -C=O(CH₂), -CH(OH)-CH₂, -CH₂-C=O, -CH₂-CH₂=CO, -CH₂-CH(OH)-CH₂, -CH₂CH(OCOR⁴)-CH₂;
R³ = 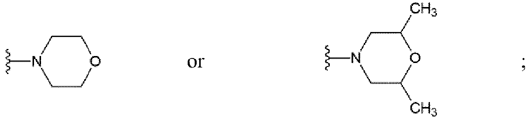 and
R⁴ = C₁-C₃ alkyl,
   , for use as a medicament, such as for use in human and/or veterinary medicine.

Preferably, R⁴ of the compound according to the invention is -CH₃. More preferably, formula (I) is 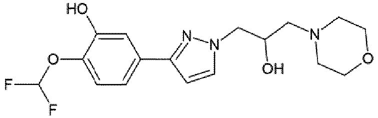 (I'). The compound of formula (I') is herein below also referred to as "RICE01" or more generally "RICE".

The invention also relates to a pharmaceutical composition comprising at least one compound according to the invention and a pharmaceutical acceptable carrier.

In yet another preferred embodiment according to the invention, the compound or composition according to the invention is used as a medicament with PDE4D inhibiting activity. Preferably, the compound, pharmaceutical composition or medicament is used in the prevention and/or treatment and/or cure of a neurodegenerative disorder such as Alzheimer's disease (AD), Parkinson's disease (PD), Huntington's disease, Lou Gehrig's disease (ALS), stroke, traumatic brain injury, spinal cord injury, mild cognitive impairment and demyelinating diseases such as peripheral demyelinating diseases, and multiple sclerosis (MS) in a subject; in particular for use in improving cognition and/or neuroplasticity and/or for the remyelination of the myelin sheet.

In yet another preferred embodiment according to the invention, the compound, pharmaceutical composition or medicament is used in the treatment of multiple sclerosis in particular progressive multiple sclerosis such as selected from the group comprising primary progressive multiple sclerosis, secondary progressive multiple sclerosis and relapse remitting multiple sclerosis.

It was found that the medicament provided an efficient and effective prevention and/or treatment and/or cure.

The pharmaceutical composition or medicament provides the same effects and advantages as those described for the compound.

For pharmaceutical use, the compounds of the invention may be used as a free acid or base, and/or in the form of a pharmaceutically acceptable acid-addition and/or base-addition salt (e.g. obtained with non-toxic organic or inorganic acid or base), in the form of a hydrate, solvate and/or complex, and/or in the form or a pro-drug or pre-drug, such as an ester. As used herein and unless otherwise stated, the term "solvate" includes any combination which may be formed by a compound of this invention with a suitable inorganic solvent (e.g. hydrates) or organic solvent, such as but not limited to alcohols, ketones, esters and the like. Such salts, hydrates, solvates, etc. and the preparation thereof will be clear to the skilled person; reference is for instance made to the salts, hydrates, solvates, etc.

The pharmaceutically acceptable salts of the compounds according to the invention, i.e. in the form of water-, oil-soluble, or dispersible products, include the conventional non-toxic salts or the quaternary ammonium salts which are formed, e.g., from inorganic or organic acids or bases.

Generally, for pharmaceutical use, the compounds of the inventions may be formulated as a pharmaceutical preparation or pharmaceutical composition comprising at least one compound of the invention and at least one pharmaceutically acceptable carrier, diluent or excipient and/or adjuvant, and optionally one or more further pharmaceutically active compounds.

By means of non-limiting examples, such a formulation or medicament may be in a form suitable for oral administration, for parenteral administration (such as by intravenous, intramuscular or subcutaneous injection or intravenous infusion), for intranasal administration etc.. Such suitable administration forms - which may be solid, semi-solid or liquid, depending on the manner of administration - as well as methods and carriers, diluents and excipients for use in the preparation thereof, will be clear to the skilled person.

Some preferred, but non-limiting examples of such preparations include tablets, pills, powders, lozenges, sachets, cachets, elixirs, suspensions, emulsions, solutions, syrups, aerosols, ointments, creams, lotions, soft and hard gelatin capsules, suppositories, eye drops, sterile injectable solutions and sterile packaged powders (which are usually reconstituted prior to use) for administration as a bolus and/or for continuous administration, which may be formulated with carriers, excipients, and diluents that are suitable per se for such formulations.

The formulations can optionally contain other pharmaceutically active substances (which may or may not lead to a synergistic effect with the compounds of the invention) and other substances that are commonly used in pharmaceutical formulations, such as lubricating agents, wetting agents, emulsifying and suspending agents, dispersing agents, desintegrants, bulking agents, fillers, preserving agents, sweetening agents, flavoring agents, flow regulators, release agents, etc.. The compositions may also be formulated so as to provide rapid, sustained or delayed release of the active compound(s) contained therein, for example using liposomes or hydrophilic polymeric matrices based on natural gels or synthetic polymers.

Particular reference is made to the compositions, formulations (and carriers, excipients, diluents, etc. for use therein), routes of administration etc.

The preparations may be prepared in a manner known per se, which usually involves mixing at least one compound according to the invention with the one or more pharmaceutically acceptable carriers, and, if desired, in combination with other pharmaceutical active compounds, when necessary under aseptic conditions.

The pharmaceutical preparations of the invention are preferably in a unit dosage form, and may be suitably packaged, for example in a box, blister, vial, bottle, sachet, ampoule or in any other suitable single-dose or multi-dose holder or container (which may be properly labeled); optionally with one or more leaflets containing product information and/or instructions for use. Generally, such unit dosages will contain between 0.005 and 1000 mg, usually between 0.01 and 1000 mg, or between 0.05 and 500 mg, of at least one compound of the invention, e.g. about 0.005, 0.01, 0.05, 1, 2.5, 5, 10, 20, 50, 100, 150, 200, 250 or 500 mg per unit dosage.

The compounds can be administered by a variety of routes including the oral, rectal, ocular, transdermal, subcutaneous, intravenous, intramuscular or intranasal routes, depending mainly on the specific preparation used and the condition to be treated or prevented, and with oral and intravenous administration usually being preferred. The at least one compound of the invention will generally be administered in an "effective amount", upon suitable administration, is sufficient to achieve the desired therapeutic or prophylactic effect in the individual to which it is administered.

Usually, depending on the condition to be prevented or treated and the route of administration, such an effective amount will usually be between 0.005 to 1000 mg per day, more often between 0.001 and 1000 mg, such as between 0.05 and 500 mg, such as for example about 0.005, 0.01, 0.05, 1, 2.5, 5, 10, 20, 50, 100, 150, 200, 250 mg or 500mg, which may be administered as a single daily dose, divided over one or more daily doses, or essentially continuously, e.g. using a drip infusion. The amount(s) to be administered, the route of administration and the further treatment regimen may be determined by the treating clinician, depending on factors such as the age, gender and general condition of the patient and the nature and severity of the disease/symptoms to be treated.

In accordance with the method of the present invention, said pharmaceutical composition can be administered separately at different times during the course of therapy or concurrently in divided or single combination forms. The present invention is therefore to be understood as embracing all such regimes of simultaneous or alternating treatment and the term "administering" is to be interpreted accordingly.

For an oral administration form, the compositions of the present invention can be mixed with suitable additives, such as excipients, stabilizers, or inert diluents, and brought by means of the customary methods into the suitable administration forms, such as tablets, coated tablets, hard capsules, aqueous, alcoholic, or oily solutions. Examples of suitable inert carriers are gum arabic, magnesia, magnesium carbonate, potassium phosphate, lactose, glucose, or starch, in particular, corn starch. In this case, the preparation can be carried out both as dry and as moist granules. Suitable oily excipients or solvents are vegetable or animal oils, such as sunflower oil or cod liver oil. Suitable solvents for aqueous or alcoholic solutions are water, ethanol, sugar solutions, or mixtures thereof. Polyethylene glycols and polypropylene glycols are also useful as further auxiliaries for other administration forms. As immediate release tablets, these compositions may contain microcrystalline cellulose, dicalcium phosphate, starch, magnesium stearate and lactose and/or other excipients, binders, extenders, disintegrants, diluents and lubricants known in the art.

For subcutaneous administration, the compound according to the invention, if desired with the substances customary therefore such as solubilizers, emulsifiers or further auxiliaries are brought into solution, suspension, or emulsion. The compounds of the invention can also be lyophilized and the lyophilizates obtained used, for example, for the production of injection or infusion preparations.

The compositions are of value in the veterinary field, which for the purposes herein not only includes the prevention and/or treatment of diseases in animals, but also - for economically important animals such as cattle, pigs, sheep, chicken, fish, etc. - enhancing the growth and/or weight of the animal and/or the amount and/or the quality of the meat or other products obtained from the animal. Thus, in a further aspect, the invention relates to a composition for veterinary use that contains at least one compound of the invention and at least one suitable carrier (i.e. a carrier suitable for veterinary use). The invention also relates to the use of a compound of the invention in the preparation of such a composition.

In another embodiment, the present invention provides a pharmaceutical composition comprising selective PDE4D inhibitor(s) as mentioned above, for use as a medicament in the diagnosis, prevention or treatment of a neurodegenerative disorder, such as Alzheimer's disease (AD), Parkinson's disease (PD), Huntington's disease, Lou Gehrig's disease (ALS), stroke, traumatic brain injury, spinal cord injury, mild cognitive impairment and demyelinating diseases such as peripheral demyelinating diseases, and multiple sclerosis (MS); in particular for use in the diagnosis, prevention and/or treatment of demyelinating diseases of the central nervous system or demyelinating diseases of the peripheral nervous system.

Alzheimer's disease (AD) is a chronic neurodegenerative disease that results in the loss of neurons and synapses in the cerebral cortex and certain subcortical structures and is associated with cognitive impairments. Alzheimer's disease pathology is primarily characterized by the presence of senile plaques and neurofibrillary tangles. Although significant research has been done in the field of Alzheimer's disease no effective treatment has been found so far.

Parkinson's disease (PD) is the second most common neurodegenerative disorder. It typically manifests as bradykinesia, rigidity, resting tremor and/or postur instability. PD is primarily characterized by death of dopaminergic neurons in the substantia nigra, a region fo the midbrain. The main known risk factor is age, although mutations in particular genes, such as SNCA, LRRK2, GBA and MAPT have also been shown to cause hereditary PD or increase PD risk.

Huntington's disease (HD) is a rare autosomal dominant neurodegenerative disorder caused by mutation in the huntingtin gene (HTT). HD is characterized by loss of medium spiny neurons and astrogliosis. The first region to be substantially affected is the stratium, followed by degeneration of the frontal and temporal cortices. Along with being a neurodegenerative disorder, HD has links to problems with neurodevelopment.

Amyotrophic lateral sclerosis (ALS), also known as Lou Gherig's disease, is a disease in which motor neurons are selectively targeted for degeneration. ALS is a neurodegenerative disorders that negatively impacts the upper motor neurons and lower motor neurons. Early diagnosis of ALS is harder than with other neurodegenerative diseases as there are no highly effective means of determining its early onset.

Stroke is a medical condition in which poor blood flow to the brain cases cell death. There are 2 main types of stroke: ischemic, due to lack of blood flow, and hemorrhagic due to bleeding. Both case parts of the brain to stop functioning properly. Signs and symptoms of a stroke may include an inability to move or feel on one side of the body, problems understanding or speaking, dizziness, or loss of vision on one side.

Traumatic brain injury, also known as an intracranial injury, is an injury to the brain caused by an external force. It can be classified based on severity, mechanism or other features. Traumatic brain injury can result is physical, cognitive, social, emotional and behavioral symptoms, and outcomes can range from complete recovery to permanent disability or death.

Spinal cord injury is damage to the spinal cord that causes temporary or permanent changes in its function. Symptoms may include loss of muscle function, sensation or autonomic function in the parts of the body served by the spinal cord below the level of the injury. Injury can occur at any level of the spinal cord and can be complete, with a total loss of sensation and muscle function at lower sacral segments, or incomplete, meaning some nervous signals are able to travel past the injured area of the cord.

Mild cognitive impairment (MCI) is a neurocognitive disorder which involves cognitive impairments beyond those expected based on an individual's age and education but which are not significant enough to interfere with instrumental activities of daily living. MCI may occur as a transitional stage between normal aging and dementia, especially Alzheimer's disease. It includes both cognitive and non-cognitive impairments.

Demyelinating diseases of the central nervous system can be multiple sclerosis, neuromyelitis optic (Devic's disease), inflammatory demyelinating diseases, central nervous system neuropathy, central pontine myelinolysis, myelopathy, leukoencephalopathy, or leukodystrophy. In a further embodiment, the demyelinating disease of the central nervous system is multiple sclerosis. In still another further embodiment, the demyelinating disease of the central nervous system is progressive multiple sclerosis. Therefore, in an even more preferred embodiment, the present invention provides a pharmaceutical composition comprising one or more selective PDE4D inhibitor(s) for use as a medicament in restoring the remyelination process in the treatment of progressive multiple sclerosis.

Multiple sclerosis (MS) is characterized by a variety of clinical symptoms, such as gradual muscle weakness, fatigue, and cognitive impairment. The destructive immunological interplay at disease onset leads to oligodendrocyte loss, focal demyelination, and axonal damage. Available therapies modulate the immune response to temper early disease activity, but have limited efficacy in preventing the transition towards the chronic stage and are no longer effective in the progressive stage of MS (pMS). Hence, there is an urgent need for therapies that halt disease progression and boost repair processes.

In still another embodiment, the demyelinating disease of the peripheral nerve system is selected from Guillain-Barré syndrome, chronic inflammatory demyelinating polyneuropathy, anti-MAG peripheral neuropathy, Charcot-Marie tooth disease, hereditary neuropathy with liability to pressure palsy; copper deficiency-associated conditions such as peripheral neuropathy, myelopathy, optic neuropathy, progressive inflammatory neuropathy, diabetic neuropathy or traumatic nerve injury. Thus, the present invention is also directed to a pharmaceutical composition comprising one or more selective PDE4D inhibitors as described above, for use in the diagnosis, prevention and/or treatment of demyelinating diseases of the peripheral nervous system; preferably demyelinating diseases of the peripheral nervous system selected from Guillain-Barré syndrome, chronic inflammatory demyelinating polyneuropathy, anti-MAG peripheral neuropathy, Charcot-Marie tooth disease, hereditary neuropathy with liability to pressure palsy; copper deficiency-associated conditions such as peripheral neuropathy, myelopathy, optic neuropathy; progressive inflammatory neuropathy, diabetic neuropathy or traumatic nerve injury.

The invention also relates to a PDE4D inhibitor comprising a compound according to the invention, wherein the PDE4D inhibitor selectively inhibits the type D isoforms of PDE4 for use in the prevention and/or treatment of a neurodegenerative disorder such as selected from the list comprising: Alzheimer's disease (AD), Parkinson's disease (PD), Huntington's disease, Lou Gehrig's disease (ALS), stroke, traumatic brain injury, spinal cord injury, mild cognitive impairment and demyelinating diseases such as peripheral demyelinating diseases, and multiple sclerosis (MS) in a subject.

The PDE4D inhibitor provides the same effects and advantages as those described for the compound and the pharmaceutical composition.

It is noted that the PDE4D inhibitor is typically characterized in that it provides selective PDE4D inhibitors for use in the treatment of neurodegenerative disorders such as selected from the list comprising: Alzheimer's disease (AD), Parkinson's disease (PD), Huntington's disease, Lou Gehrig's disease (ALS), stroke, traumatic brain injury, spinal cord injury, mild cognitive impairment and demyelinating diseases such as peripheral demyelinating diseases, and multiple sclerosis (MS).

In contrast to pan-PDE4 inhibitors that inhibit all types of isoforms of PDE4D at a high level, the present invention is directed to selective PDE4D inhibitors that selectively inhibit the type D isoform of PDE4. In the context of the present invention, selective inhibition of the type D isoforms of PDE4 is defined as at least 50% inhibition of the activity of the type D isoforms of PDE4 and maximum 45% inhibition of the activity of the other (type A, B and C) isoforms of PDE4. In an even preferred embodiment, selective inhibition of the type D isoform of PDE4 is defined as at least 60% inhibition of the activity of the type D isoforms of PDE4 and maximum 45% inhibition of the activity of the other (type A, B and C) isoforms of PDE4. Thus, in the context of the present invention, selective PDE4D inhibitors are inhibitors that inhibit at least 50% of the activity of type D isoforms of PDE4 and inhibit the activity of the other Type A, B and C isoforms of PDE4 with maximum 45%.

It was found that selective PDE4D inhibitors, such as for example RICE (see details in examples part), boosted OPC differentiation in primary OPCs in vitro. Furthermore, it was found that selective PDE4D inhibition improved (re)myelination in ex vivo demyelinated cerebellar brain slices. It is noted that RICE or RICE01 may refer to RICE3, RICE10, RICE30, RICE100, RICE300, and/or RICE1000, wherein the number refers to the concentration (nM) of the compound as defined in the examples part.

The inventors surprisingly found that selective PDE4D inhibitors, such as for example RICE (compound according to the invention), stimulated the differentiation of oligodendrocytes *in vitro* and improved (re)myelination in *ex vivo* demyelinated cerebellar brain slices, thereby rendering them suitable in preventing the transition towards the progressive stage when cognitive impairment features. Remarkably, and in contrast to the pan-PDE4 inhibitors, such as roflumilast, only low doses of the selective PDE4D inhibitors are sufficient to achieve their effect, and hence, emetic side-effects, that are often observed after treatment with pan-PDE4 inhibitors, are expected to be absent. The present invention is therefore directed to selective PDE4D inhibitor(s) that selectively inhibit the type D isoform of PDE4 for use in the prevention and/or treatment of neurodegenerative disorders such as selected from the list comprising: Alzheimer's disease (AD), Parkinson's disease (PD), Huntington's disease, Lou Gehrig's disease (ALS), stroke, traumatic brain injury, spinal cord injury, mild cognitive impairment and demyelinating diseases such as peripheral demyelinating diseases, and multiple sclerosis (MS).

In a further embodiment, the selective PDE4D inhibitor is for use in restoring the remyelination process in the treatment of neurodegenerative disorders, in particular demyelinating diseases of the central nervous system.

In yet another preferred embodiment according to the invention, the invention is directed to said selective PDE4D inhibitors for use in the prevention and/or treatment of multiple sclerosis, wherein the selective PDE4D inhibitor(s) restore the remyelination process in the treatment of MS of said subject.

In another embodiment, the selective PDE4D inhibitor(s) for use according to the invention, restore the remyelination process in the treatment of progressive MS (pMS) of said subject, it is accordingly an objective of the present invention to provide selective PDE4D inhibitor(s) for use in the prevention and/or treatment and/or curing of progressive MS in a subject, more in particular for use in the prevention and/or treatment of primary progressive multiple sclerosis, secondary progressive multiple sclerosis or relapse remitting multiple sclerosis.

As used herein, the term "demyelinating disease", is a disease condition in which the myelin sheath which surrounds neurons in nervous tissue is lost or damaged, leading to axonal degeneration and impaired signal transduction in the affected nerves. A demyelinating disease of the central nervous system is a disease in which the myelin sheaths of neurons in the central nervous system are lost or damaged. Examples of demyelinating diseases of the central nervous systems are multiple sclerosis, neuromyelitis optic (Devic's disease), inflammatory demyelinating diseases, central nervous system neuropathy, central pontine myelinolysis, myelopathy, leukoencephalopathy, or leukodystrophy.

A demyelinating disease of the peripheral nervous system is a disease condition in which the myelin sheaths of neurons in the peripheral nervous system are lost or damaged. Examples of demyelinating diseases of the peripheral nervous system are Guillain-Barré syndrome, chronic inflammatory demyelinating polyneuropathy, anti-MAG peripheral neuropathy, Charcot-Marie tooth disease, hereditary neuropathy with liability to pressure palsy; copper deficiency-associated conditions such as peripheral neuropathy, myelopathy, optic neuropathy; progressive inflammatory neuropathy, diabetic neuropathy or traumatic nerve injury.

As used herein, the term "multiple sclerosis" or "MS" entails an autoimmune-mediated process in which an abnormal response of the body's immune system is directed against the central nervous system (CNS), which is made up of the brain, spinal cord and optic nerves. The immune reaction results in death of oligodendrocytes, demyelination, and eventually loss of axons, featured by a physical and cognitive disability.

As used herein, the term "progressive multiple sclerosis" or "pMS" is featured by an accumulation of chronic demyelinated lesions and is subdivided in Primary progressive MS (PPMS), Secondary progressive MS (SPMS) and relapse remitting MS (RRMS).

Primary progressive MS (PPMS) is characterized by worsening neurologic function (accumulation of disability) from the onset of symptoms, without early relapses or remissions. PPMS can be further characterized at different points in time as either active (with an occasional relapse and/or evidence of new MRI activity) or not active, as well as with progression (evidence of disease worsening on an objective measure of change over time, with or without relapse or new MRI activity) or without progression.

Secondary progressive MS (SPMS) follows an initial relapsing-remitting course. Most people who are diagnosed with a relapse remitting MS (RRMS) will eventually transition to a secondary progressive course in which there is a progressive worsening of neurologic function (accumulation of disability) over time. SPMS can be further characterized at different points in time as either active (with relapses and/or evidence of new MRI activity) or not active, as well as with progression (evidence of disease worsening on an objective measure of change over time, with or without relapses) or without progression.

In a preferred embodiment according to the invention, the subject may be a non-human animal or a human. Preferably, the selective PDE4D inhibitor is administered at a dose rate between 0.005 and 1000 mg, such as between 0.01 mg and 1000 mg, preferably at a dose rate between 0.025 mg and 750 mg, more preferably at a dose rate between 0.05 mg and 500 mg.

Furthermore, the invention provides the use of selective PDE4D inhibitors in *in vitro, ex vivo* and *in vivo* remyelination assays.

Said in vitro, ex vivo and in vivo remyelination assays may for example be characterized by OPC differentiation assays *(in vitro),* brain slices (*ex vivo*) and cuprizone modelling with a molecular and functional readout (*in vivo*).

Selective PDE4D inhibitory compounds for use in the aforementioned indications can be identified using a PDE4 inhibition assay known in the art. PDE4D inhibition assays can be performed for example using recombinant human PDE enzymes expressed in a baculoviral system. The preliminary screening assays can be performed by the IMAP technology (Molecular Devices), which is based on the high affinity binding of phosphate by immobilized metal coordination complexes on nanoparticles. The binding reagent complexes with phosphate groups on nucleotide monophosphate generated from cyclic nucleotides (cAMP) through phosphodiesterases. With fluorescence polarization detection, binding causes a change in the rate of the molecular motion of the phosphate bearing molecule and results in an increase in the fluorescence polarization value observed for the fluorescent label attached to the substrate. Rolipram can be used as reference compound. All compounds can be solved in DMSO at 10⁻² M concentration and then diluted with water to the final suitable concentrations. All synthesized compounds can be tested preliminary on PDE4D at 10⁻⁵ M concentration, in duplicate. Results showing an inhibition of the control higher than 50% are considered to represent significant effects of the test compounds. IC₅₀ values of less than 10 µM are considered to be potent PDE4 inhibitors.

Compounds showing inhibition control higher than 50% on PDE4D can be further tested on the same isoform enzyme at five concentrations in the interval 10⁻⁸-10⁻⁴ M. IC₅₀ values for rolipram and tested compounds can be determined by nonlinear regression analysis of its inhibition curve, using Hill equation curve fitting (Graph Pad Prism software). IC₅₀ values are reported at µM concentration.

Said inhibition may be effected *in vitro, ex vivo* and/or *in vivo,* and when effected *in vivo,* is preferably effected in a selective manner, as defined above.

The invention also relates to a method for preventing and/or treating and/or curing a neurodegenerative disorder such as selected from the list comprising: Alzheimer's disease (AD), Parkinson's disease (PD), Huntington's disease, Lou Gehrig's disease (ALS), stroke, traumatic brain injury, spinal cord injury, mild cognitive impairment and demyelinating diseases such as peripheral demyelinating diseases, and multiple sclerosis (MS), comprising administering a pharmaceutical composition according to the invention.

The method for preventing and/or treating and/or curing a demyelinating disease provides the same effects and advantages as those described for the compound, the pharmaceutical composition, and PDE4D inhibitor.

The method according to the invention for preventing and/or treating and/or curing neurodegenerative disorders, such as selected from the list comprising: Alzheimer's disease (AD), Parkinson's disease (PD), Huntington's disease, Lou Gehrig's disease (ALS), stroke, traumatic brain injury, spinal cord injury, mild cognitive impairment and demyelinating diseases such as peripheral demyelinating diseases, and multiple sclerosis (MS), in particular demyelinating diseases of the central or peripheral nervous system, comprises administering a pharmaceutical composition according to the invention to said subject.

In a further embodiment, the present invention provides a method for preventing and/or treating and/or curing multiple sclerosis, preferably progressive multiple sclerosis in a subject. Said prevention and/or treatment and/or cure comprise administering a pharmaceutical composition according to the invention to said subject. In an even further embodiment, the present invention provides a method for restoring the remyelination process in the treatment of progressive multiple sclerosis in a subject, said method comprising administering a pharmaceutical composition according to the invention to said subject.

In yet another embodiment, the present invention provides a method for preventing and/or treating and/or curing demyelinating diseases of the peripheral nervous system, preferably demyelinating diseases of the peripheral nervous system associated with peripheral neuropathy. In an even more preferred embodiment, the demyelinating disease of the peripheral nervous system is selected from Guillain-Barré syndrome, chronic inflammatory demyelinating polyneuropathy, anti-MAG peripheral neuropathy, Charcot-Marie tooth disease, hereditary neuropathy with liability to pressure palsy, copper deficiency-associated conditions such as peripheral neuropathy, myelopathy, optic neuropathy; progressive inflammatory neuropathy or traumatic nerve injury.

The invention also relates to a method for synthesis of a compound according to the invention, comprising the steps of:
- providing a compound according to formula (II); 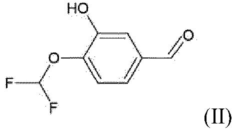
- reacting the compound according to formula (II) with 4-Methoxybenzyl chloride (PMBCl) forming a compound according to formula (III); 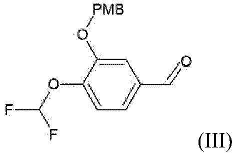
- dissolving the compound according formula (III) in acetonitrile (MeCN);
- reacting the dissolved compound according to formula (III) to form the compound according to formula (IV), 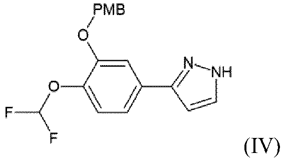 wherein the step of reacting comprises:
   - adding toluene-4-sulfonic acid hydrazide to the dissolved compound according to formula (III) forming a reaction mixture;
   - adding sodium hydroxide (NaOH) to the reaction mixture; and
   - adding 1-vinlyimidazole to the reaction mixture;
- reacting the compound according formula (IV) with 2-(chloromethyl)oxirane and trimethylamine (Et₃N) to form the compound according to formula (V); 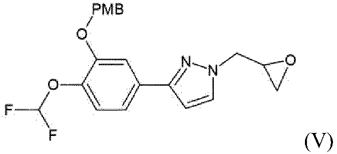
- reacting the compound according formula (V) with morpholine to form the compound according to formula (VI); and 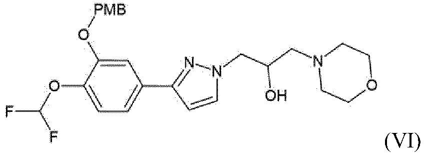
reacting the compound according to formula (VI) with trifluoroacetic acid (TFA) to form the compound according to formula (I').

The method for synthesis of a compound according to the invention provides the same effects and advantages as those described for the compound, the pharmaceutical composition, PDE4D inhibitor, and method for preventing and/or treating and/or curing a neurodegenerative disorder such as selected from the list comprising: Alzheimer's disease (AD), Parkinson's disease (PD), Huntington's disease, Lou Gehrig's disease (ALS), stroke, traumatic brain injury, spinal cord injury, mild cognitive impairment and demyelinating diseases such as peripheral demyelinating diseases, and multiple sclerosis (MS).

Preferably, the step of adding toluene-4-sulfonic acid hydrazide to the dissolved compound according to formula (III) forming a reaction mixture is performed at a temperature in the range of 15 °C to 40 °C, preferably at a temperature in the range of 15 °C to 30 °C, more preferably at a temperature of about 20°C.

In yet another preferred embodiment according to the invention, the step of adding toluene-4-sulfonic acid hydrazide to the dissolved compound according to formula (III) forming a reaction mixture is performed for at least 45 minutes, more preferably performed for at least 55 minutes, more preferably performed for about 60 minutes. Preferably, the step of adding sodium hydroxide (NaOH) to the reaction mixture is performed for at least 10 minutes, preferably for at least 15 minutes, more preferably for at least 20 minutes.

It is noted that potassium hydroxide or any other suitable hydroxide may be used instead of the sodium hydroxide.

In yet another preferred embodiment according to the invention, the step of reacting the compound according formula (V) with morpholine to form the compound according to formula (VI) is performed at a temperature in the range of 30 °C to 80 °C, preferably performed at a temperature in the range of 40 °C to 70 °C, more preferably performed at a temperature in the range of 50 °C to 60 °C.

The term "alkyl" by itself or as part of another substituent refers to a fully saturated hydrocarbon of Formula CₓH₂ₓ₊₁ wherein x is a number greater than or equal to 1. Generally, alkyl groups of this invention comprise from 1 to 20 carbon atoms. Alkyl groups may be linear or branched and may be substituted as indicated herein. When a subscript is used herein following a carbon atom, the subscript refers to the number of carbon atoms that the named group may contain. Thus, for example, C₁-C₄ alkyl means an alkyl of one to four carbon atoms. Examples of alkyl groups are methyl, ethyl, n-propyl, i-propyl, butyl, and its isomers (e.g. n-butyl, i-butyl and t-butyl); pentyl and its isomers, hexyl and its isomers, heptyl and its isomers, octyl and its isomers, nonyl and its isomers; decyl and its isomers. C₁-C₆ alkyl includes all linear, branched, or cyclic alkyl groups with between 1 and 6 carbon atoms, and thus includes methyl, ethyl, n-propyl, i-propyl, butyl and its isomers (e.g. n-butyl, i-butyl and t-butyl); pentyl and its isomers, hexyl and its isomers, cyclopentyl, 2-, 3-, or 4-methylcyclopentyl, cyclopentylmethylene, and cyclohexyl.

The term "optionally substituted" refers to a certain group optionally substituted with one or more substituents (for example 1 to 4 substituents, for example 1, 2, 3, or 4 substituents or 1 to 2 substituents) at any available point of attachment.

### BRIEF DESCRIPTION OF THE DRAWINGS

Further advantages, features and details of the invention are elucidated on the basis of preferred embodiments thereof, wherein reference is made to the accompanying drawings, in which:
- Figure 1 shows OPC differentiation (*in vitro*);
- Figure 2 shows myelination in brain slices (*ex vivo*);
- Figure 3 shows visual evoked potentials (*in vivo*); and
- Figure 4 shows neurite outgrowth *(in vitro).*
- Figure 5 shows differentiation of a human oligodendrocyte cell line *(in vitro)*
- Figure 6 shows PDGFRa and MBP expression in a mouse cell line (*in vitro*)
- Figure 7 shows neurite length in a mouse neuroblastoma cell line (*in vitro*)
- Figure 8 shows discrimination index in the object location task (*in vivo*)

### EXAMPLES

The present invention is by no means limited to the above described preferred embodiments and/or experiments thereof. The rights sought are defined by the following claims within the scope of which many modifications can be envisaged.

In the presentation of the below experimental results and figures, the specific PDE4D inhibitor RICE is used herein as "RICE01, RICE3, RICE10, RICE30, RICE100, RICE300, and/or RICE1000" - depending on the tested concentrations in-between 3 nM and 1000 nM. All abbreviations are used and refer to the same inhibitor RICE. Unless specifically stated that RICE01, RICE3, RICE10, RICE30, RICE100, RICE300, and/or RICE1000 is mentioned.

The pan-PDE4 inhibitor roflumilast is sometimes abbreviated as 'Roflu'. Both the terms "roflumilast" and "roflu" are used and refer to the same inhibitor roflumilast.

### Example 1: OPC differentiation (in vitro)

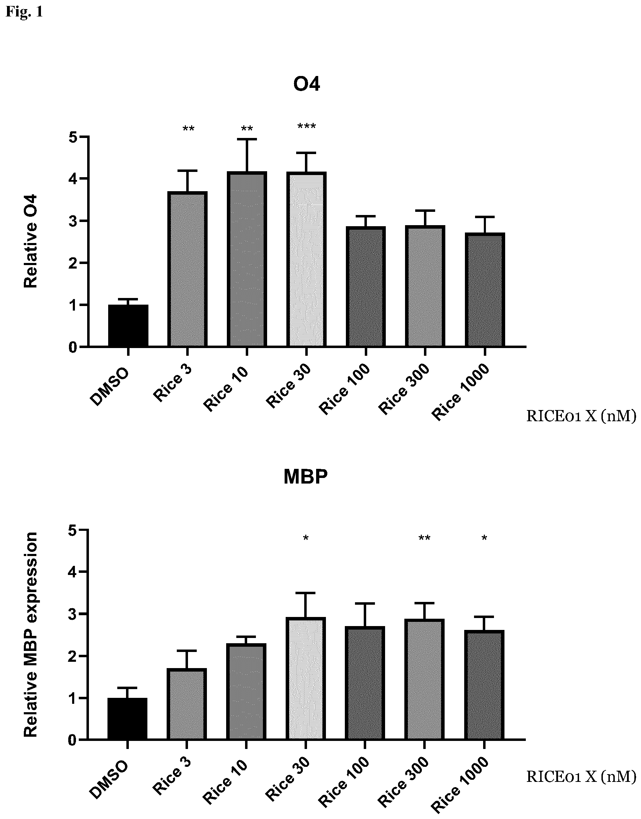

Primary mouse oligodendrocyte precursor cells (OPCs) were isolated from p0 C57bl6 pups using the shake off method at day 0 (Figure 1). Figure 1 shows OPC differentiation (*in vitro*) wherein the x-axis represents the PDE4D inhibitor (from left to right DMSO, RICE3, RICE10, RICE30, RICE100, RICE300, RICE1000) and the y-axis the relative O4. Primary OPCs (150.000 cells/condition) were cultured and stimulated with vehicle (0.1% DMSO) or different concentration of RICE (3 nM, 10 nM, 30 nM, 100 nM, 300 nM, 1000 nM) in 0.1% DMSO. Treatment was repeated on day 2 and day 4, applying a 40% medium change. Cells were fixated at day 6 and stained for O4, a late OPC marker, and MBP, an oligodendrocyte marker. The O4 expression increased with the lower concentrations of RICE, reaching the maximal expression increase at 10 nM of treatment. The increased expression of O4 declined from 100 nM RICE onwards. The MBP expression dose dependently increased, reaching it's maximal expression at 30 nM of treatment. These data together indicate that nanomolar ranges of RICE boosts OPC differentiation towards oligodendrocytes. Data are displayed as mean +/-SEM and were analyzed using a non-parametric Kruskal-Wallis test, *p<0,05; **p<0,01, ***p<0,005)(n≥5/group).

### Example 2: Myelination in brain slices (ex vivo)

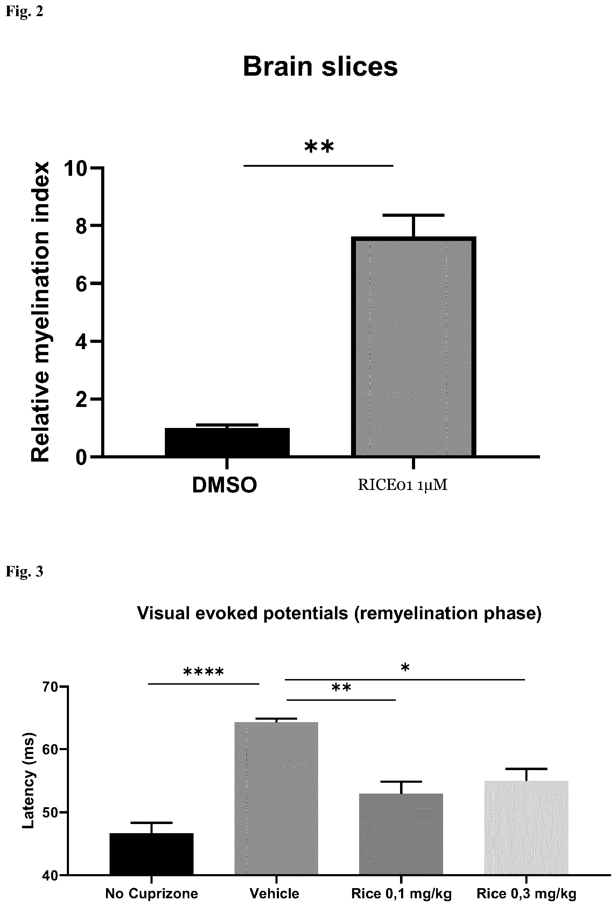

Mouse cerebellar brain slices (350µm) were made from the cerebellum of p10 C57bl6 pups. After 4 days in culture, slices were demyelinated using 0,5mg/ml lysolecithin (18h) and subsequently treated for 10 days with 1µM RICE (in 0,1% DMSO) (Figure 2). Figure 2 shows on the x-axis the PDE4D inhibitor (from left to right DMSO and RICE (1µM) and the y-axis the relative myelination index or relative MBP expression. Treatment was repeated every 2 days with a 50% medium replenishment. After 10 days of treatment, brain slices were fixated using 4% paraformaldehyde and stained for MBP and neurofilament. Images (z-stacks) were taken with the Zeiss LSM880 confocal microscope on the same day. Three slices per animal were quantified for their myelination index (colocalisation volume between MBP and neurofilament, corrected for the total pressence of neurofilament in the image). Data shown are displayed as mean +/- SEM and analyzed using the non-parametric Mann-Whitney test (**p<0,01).

### Example 3: Visual evoked potentials (in vivo)

Male wild type C57bl6 mice were fed regular chow (n=6) or 0,3% (w/w)cuprizone (n= 21) for 6 weeks to induce remyelination. Afterwards, cuprizone was removed and recovery was allowed. Two days before ceasing the cuprizone diet, until the end of the expeirment, animals received twice a day subcutaneous injections consisting of 0,1% DMSO in 0,5% methylcellulose solution with either vehicle (no cuprizone and vehicle group; n=6 and n=8 respectively), 0,1 mg/kg RICE (n=7) or 0,3 mg/kg RICE (n=6) (Figure 3). Figure 3 shows visual evoked potentials (remyelination phase), wherein the x-axis shows from left to right no cuprozine, vehicle, RICE 0.1 mg/kg, and RICE 0.3 mg/kg, and the y-axis shows latency (ms).

After either three or four days after ceasing the cuprizone diet, visual evoked potential were measured to evaluate optic tract and nerve signaling (groups equally devided over testing days). Epidermal VEP was recorded in response to flash stimulation (3 trains of 20 stimuli, 10µs duration, 1 Hz frequency) from both eyes.

After cuprizone administration, the vehicle treated group showed significantly increased latency time, indicating damage at the myelin sheath of the optic nerve. The RICE treated groups showed decreased latency times indicating increased levels of remyelination compared to the vehicle treated group. Data shown are displayed as mean +/- SEM. A one-way ANOVA with Kruskall Wallis multiple comparison test was conducted to evaluate statistical significances (*p≤0,05; **p<0,01, ****p<0,001).

### Example 4: Neurite outgrowth (in vitro)

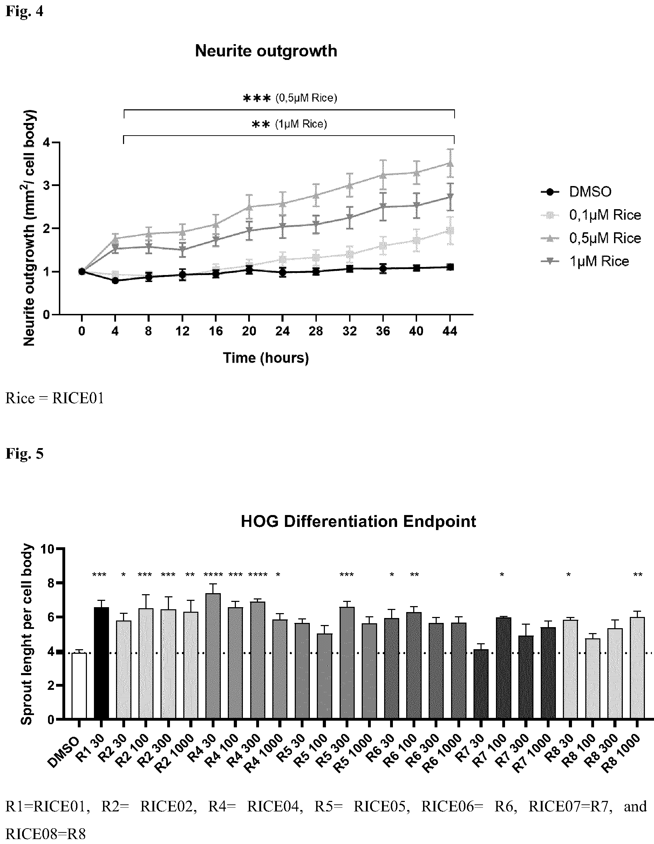

The mouse neuronal celline N2a was plated in a 96-well plate at a density of 3000 cells/well (Figure 4). Figure 4 shows on the x-axis time (hours) and on the y-axis neurite outgrowth (mm²/cell body), wherein from bottom to top at time is 44 hours the graph represents DMSO, 0.1µM RICE, 0.5 µM RICE, 1 µM RICE. One hour after plating, cells were treated with 0,1% DMSO or different concentrations of RICE (0,1µM, 0,5µM or 1µM) in 0,1% DMSO. Afterwards, cells were placed in the incucyte and the Neurotrack automated neurite outgrowth track and analysis was conducted. For this, 9 pictures were taken every 4 hours for each well and the neurite length was plotted in time for each condition (n≥4/group). Two way ANOVA analysis was conducted with Dunnett's multiple comparisons indicating a significant difference for each time point for the 0,5µM and 1µM RICE conditions compared to the vehicle. Data are shown as mean +/- SEM (**p>0,01; ***p>0,005).

### Examples 5-8

As from hereon, further to RICE01 as defined above, several other compounds falling within the scope of the present invention have been synthesized and tested (RICE02 - RICE08). A similar annotation as used herein above for RICE01 is used, for example R2 100 is meant to be RICE02 tested at a concentration of 100 nM. The synthesis schemes for each of these compounds may be found at the end of the EXAMPLES part.

### Example 5

Figure 5 shows that RICE treatment induces differentiation of a human oligodendrocyte cell line, indicative of remyelination-inducing properties of RICE compounds.

Human oligodendrocytes (HOG - celline) were plated in a 96 well plate (5.000 cells/well). Cells were cultured for 48h with treatment starting 4h post plating the cells. Cells were cultured and stimulated with vehicle (0.1% DMSO) or RICE compounds (30nM RICE01 or 30-1000nM RICE02-08). Phase-contrast pictures were taken every 3h using the Incucyte and the level of of complexity of treated HOG cells was determined using the NeuroTrack analysis. Data (n≥6/group) are displayed as mean +/-SEM. Data were analyzed using a one-way ANOVA with Dunnett's multiple comparison test (compared to DMSO condition) (*p≤0.05; **p≤0.01; ***p≤0.005; ****p≤0.001)

### Example 6

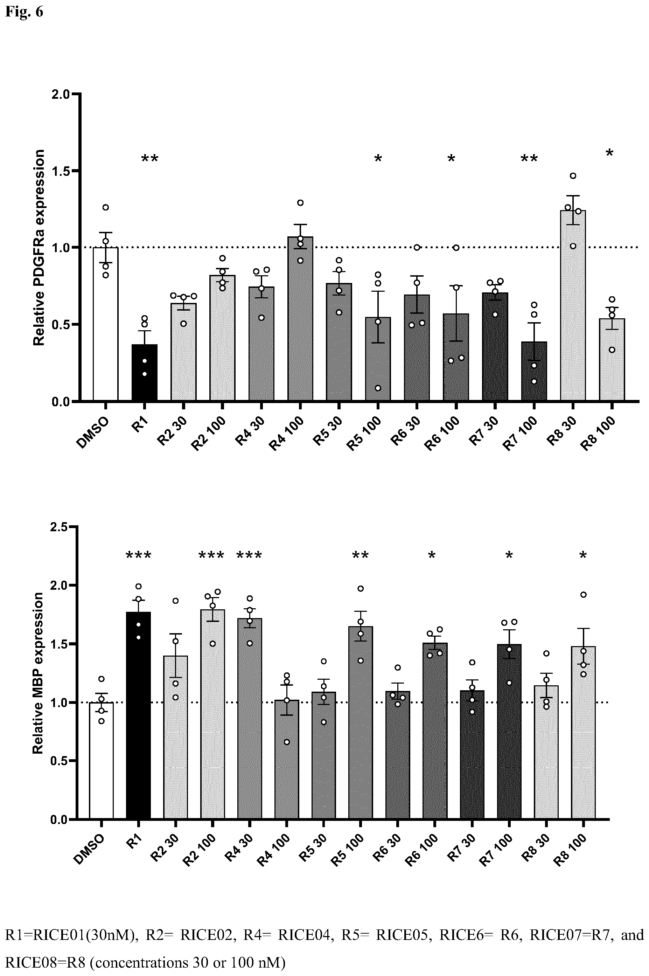

Figure 6 shows that RICE treatment decreases PDGFRa and increases MBP expression in a mouse cell line, supporting remyelination-inducing properties of RICE compounds (e.g. decrease in PDGFRa combined with an increase in MBP expression.

Mouse OPCs (Olineu-celline) were plated in a 24 well plate (100.000 cells/well). Cells were cultured for 72h with treatment starting 4h post plating the cells and was repeated after 48h. Cells were cultured and stimulated with vehicle (0.1% DMSO) or RICE compounds (30nM RICE01 or 30/100nM RICE02-08). After 72h, cells were lysated with Qiazol, after which RNA was extracted and cDNA was synthesized. Gene expression of PDGFRa (OPC marker) and MBP (oligodendrocytes marker) was determined using real-time qPCR. Data were normalized for the most stable housekeeping genes (YWHAZ and ACTB). Data (n=4/group) are displayed as mean +/-SEM. Data were analyzed using a one-way ANOVA with Dunnett's multiple comparison test (compared to DMSO condition) (*p≤0.05; **p≤0.01)

### Example 7

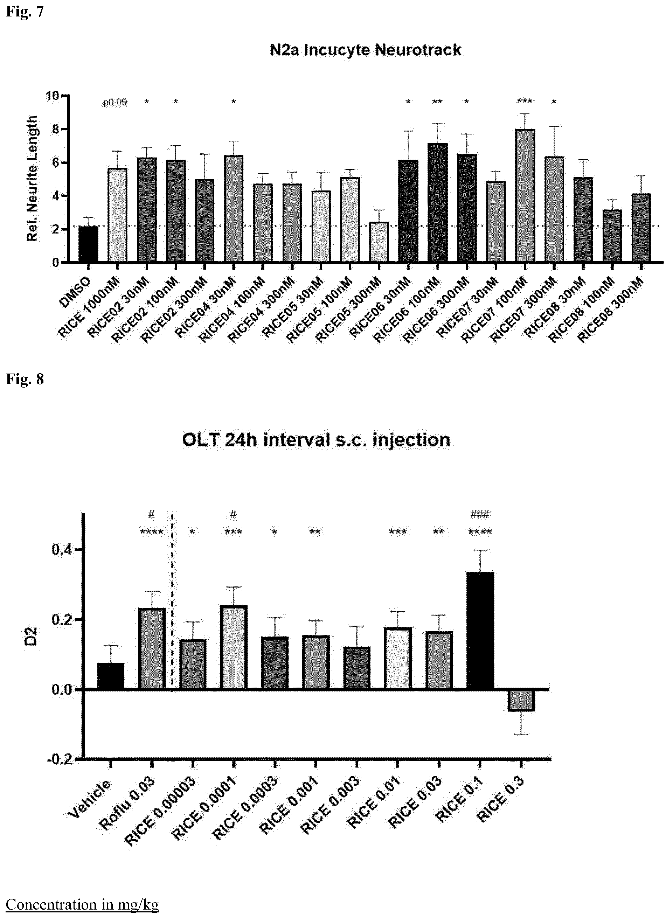

Figure 7 shows that RICE treatment increases neurite length in a mouse neuroblastoma cell line, indicating neuroplasticity-inducing capacities of RICE compounds.

Mouse N2a cells were plated in a 96 well plate at 1500 cells/well in DMEM with 2% FCS and 1% pen/strep. After 1 hour, the cells were treated with vehicle (0.1% DMSO) or RICE compounds (RICE01 1000nM or RICE02-08 30nM-300nM ) and placed in the Incucyte live cell imager (Sartorius). Phase images at 20x magnification were taken every 3 hours for 48 hours in total. Incucyte NeuroTrack analysis was performed to determine neurite length per square mm. 48h endpoint measurements were normalized against timepoint zero. Data (n=6/group) are displayed as mean +/-SEM. Data were analyzed using a one-way ANOVA with Dunnett's multiple comparison test (compared to DMSO condition) (*p≤0.05; **p≤0.01)

### Example 8

Figure 8 shows that RICE01 increases discrimination index (D2) in the object location task (OLT) with a 24h intertrial interval in young adult C57BL/6J-OlaHsd mice. These data show spatial memory promoting properties in mice (cognition enhancement)

Mice received a subcutaneous injection with vehicle (0.1% DMSO in 2% Tween80 and 0.5% methylcellulose), roflumilast 0.03 mg/kg as a positive control, or RICE01 (0.00003mg/kg - 0.3 mg/kg) 3h after the first learning trial of the 24h interval OLT. Animals exploring less that 4 seconds in either T1 or T2 were excluded from the analysis since this does not allow to reliably measure memory performance. Data (n=15-24/group) are displayed as mean +/-SEM. A onesample t-test revealed that the D2 value in 0.00003mg/kg, 0.0001mg/kg, 0.0003mg/kg, 0.001 mg/kg, 0.01mg/kg, 0.03mg/kg, and 0.1 mg/kg were significantly higher than zero, indicating discrimination between object locations (*: *p* < 0.05, **: *p <* 0.01 ***: *p* < 0.001, ****: *p* < 0.0001). When compared with vehicle treatment, RICE 0.0001mg/kg and 0.1mg/kg had a significantly higher d2 index, as assessed with Dunnett's multiple comparison test: #: *p* < 0.05, ###: *p <* 0.001).

### Synthesis routes of further compounds of the invention

Further to the specific compound RICE as defined herein above, several variant compounds have been made of which the synthesis routes and if available experimental data can be found herein below.

### RICE02

### General reaction scheme

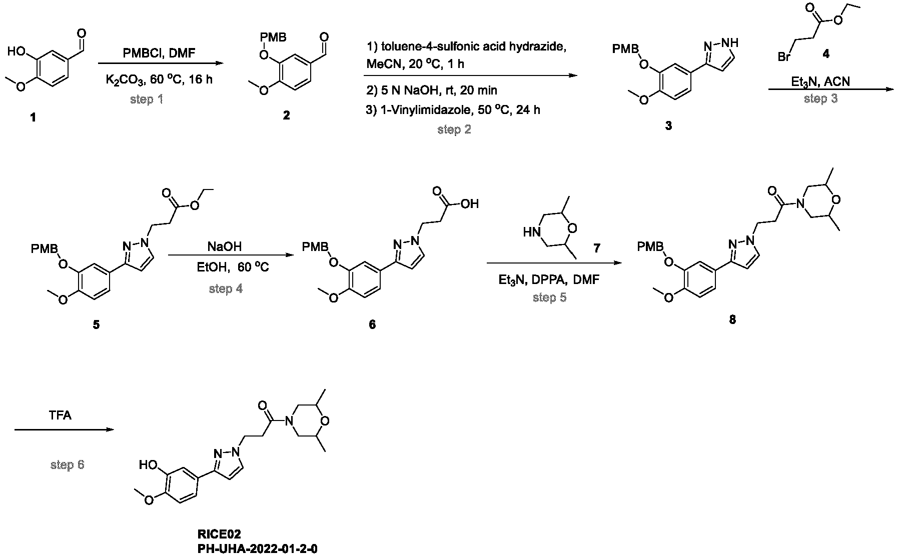

### Synthesis of 4-methoxy-3-((4-methoxybenzyl)oxy)benzaldehyde

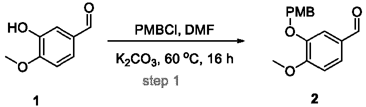

To a stirred solution of 3-hydroxy-4-methoxybenzaldehyde (15 g, 98.588 mmol, 1 equiv) and K₂CO₃ (17.71 g, 128.164 mmol, 1.3 equiv) in DMF (150 mL) was added PMBCl (17.14 g, 109.433 mmol, 1.11 equiv) in portions and the mixture was stirred for 16 h at 60 °C. The resulting mixture was filtered. The filtrate was concentrated under reduced pressure to give the crude product. The crude product was purified by silica gel column chromatography eluted with EtOAc/PE (1:1) to afford 4-methoxy-3-((4-methoxybenzyl)oxy)benzaldehyde (23 g, 59.97%) as a white solid. LC/MS: 273 [M+H]⁺

### Synthesis of 3-(4-methoxy-3-((4-methoxybenzyl)oxy)phenyl)-1H-pyrazole

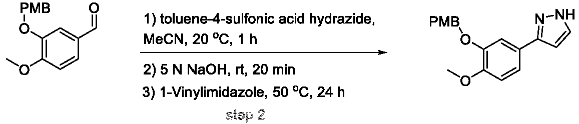

To a solution of 4-methoxy-3-[(4-methoxyphenyl)methoxy]benzaldehyde (23 g, 84.466 mmol, 1 equiv) in MeCN (150 mL) was added toluene-4-sulfonic acid hydrazide (16.52 g, 88.689 mmol, 1.05 equiv) in portions and stirred for 1 h at room temperature. Then NaOH (20 mL, 5 M) was added to the above mixture. The mixture was stirred for 20 min at the same temperature. After that, 1-vinylimidazole (39.75 g, 422.330 mmol, 5 equiv) was slowly added to the above mixture and the mixture was stirred for 24 h at 50 °C. After cooling to room temperature, the solvent was removed under reduced pressure and the residue was dissolved in ethyl acetate (100 mL). The organic layers were washed with brine (100 mL), 1 N HCl solution (51 mL) and water (51 mL), dried over anhydrous Na2SO4. After filtration, the filtrate was concentrated under reduced pressure to give the crude product. The crude product was purified by silica gel column chromatography, eluted with EtOAc/PE (1:3) to afford 3-(4-methoxy-3-((4-methoxybenzyl)oxy)phenyl)-1H-pyrazole (23 g, 43.87%) as a yellow oil. LC/MS: 311 [M+H]⁺.

### Synthesis of ethyl 3-(3-(4-methoxy-3-((4-methoxybenzyl)oxy)phenyl)-1H-pyrazol-1-yl)propanoate

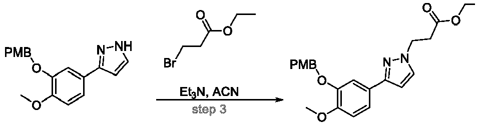

To a mixture of 3-(4-methoxy-3-((4-methoxybenzyl)oxy)phenyl)-1H-pyrazole (12 g, 38.666 mmol, 1 equiv) and ethyl 3-bromopropanoate (42.00 g, 231.996 mmol, 6 equiv) was added TEA (10.29 g, 101.692 mmol, 2.63 equiv) dropwise at 0 °C. Then NaI (2.90 g, 19.333 mmol, 0.5 equiv) was added. The resulting mixture was stirred for 3 h at 120 °C. After cooling to room temperature, to the resulting mixture was added water (500 mL). The mixture was extracted with EtOAc (3 x 500mL). The combined organic layers were washed with water (3 x 500 mL), dried over anhydrous Na₂SO₄. After filtration, the filtrate was concentrated under reduced pressure. The residue was purified by silica gel column chromatography, eluted with EtOAc/PE (1:3) to afford ethyl 3-(3-(4-methoxy-3-((4-methoxybenzyl)oxy)phenyl)-1H-pyrazol-1-yl)propanoate (8.5 g, 32.13%) as a light yellow oil. LC/MS: 411 [M+H]⁺.

### 4. Synthesis of 3-(3-(4-methoxy-3-((4-methoxybenzyl)oxy)phenyl)-1H-pyrazol-1-yl)propanoic acid

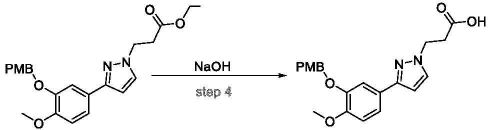

To a mixture of ethyl 3-(3-(4-methoxy-3-((4-methoxybenzyl)oxy)phenyl)-1H-pyrazol-1-yl)propanoate (8.5 g, 20.708 mmol, 1 equiv) in EtOH (96%) (50 mL) and was added NaOH (3.31 g, 82.832 mmol, 4 equiv). The mixture was stirred at 60°C for 2 h. The mixture was allowed to cool down to 25 °C. The resulting mixture was concentrated under reduced pressure. The residue was diluted with water (200 mL). The pH was adjusted to 2-3 with 1N HCl solution. The resulting solution was extracted with ethyl acetate (3x 200 mL). The combined organic layers were dried over anhydrous sodium sulfate and concentrated under reduced pressure. The residue was applied onto a silica gel column and eluted with DCM/MEOH(10:1) to give 3-(3-(4-methoxy-3-((4-methoxybenzyl)oxy)phenyl)-1H-pyrazol-1-yl)propanoic acid (4.5 g, 25.57%) as a yellow oil. LC/MS: 383 [M+H]⁺.

### 5. Synthesis of 1-(2,6-dimethylmorpholino)-3-(3-(4-methoxy-3-((4-methoxybenzyl)oxy)phenyl)-1H-pyrazol-1-yl)propan-1-one

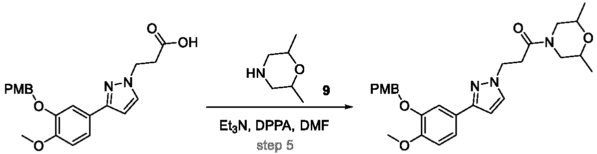

To a mixture of 3-(3-(4-methoxy-3-((4-methoxybenzyl)oxy)phenyl)-1H-pyrazol-1-yl)propanoic acid (4.5 g, 11.767 mmol, 1 equiv) in DMF (20 mL) were added TEA (1.78 g, 17.6 mmol, 1.5 equiv) dropwise at 0 °C and then 2,6-dimethylmorpholine (2.71 g, 23.534 mmol, 2 equiv), DPPA (4.05 g, 14.709 mmol, 1.25 equiv). The resulting mixture was stirred for 15 h at 80 °C. After cooling to room temperature, the resulting mixture was poured into water (200 mL). The mixture was extracted with EtOAc (2 x 200 mL). The combined organic layers were washed with water (2 x 200 mL), dried over anhydrous Na₂SO₄. After filtration, the filtrate was concentrated under reduced pressure. The residue was purified by silica gel column chromatography, eluted with EtOAc/PE (1:1) to afford 1-(2,6-dimethylmorpholino)-3-(3-(4-methoxy-3-((4-methoxybenzyl)oxy)phenyl)-1H-pyrazol-1-yl)propan-1-one (1 g, 10.63%) as a light yellow oil. LC/MS: 480 [M+H]⁺.

### 6. Synthesis of 1-(2,6-dimethylmorpholino)-3-(3-(3-hydroxy-4-methoxyphenyl)-1H-pyrazol-1-yl)propan-1-one

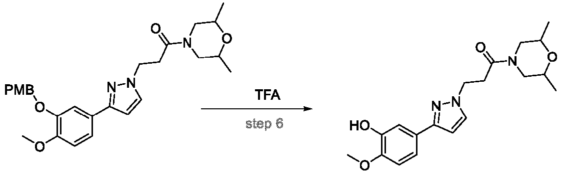

To a solution of 1-(2,6-dimethylmorpholino)-3-(3-(4-methoxy-3-((4-methoxybenzyl)oxy)phenyl)-1H-pyrazol-1-yl)propan-1-one (190 mg, 0.251 mmol, 1 equiv) was added TFA (2 mL). The resulting mixture was stirred for 2 h at 25 °C. The reaction was quenched with water (3 x 30 mL). The resulting mixture was extracted with EtOAc (3 x 30 mL). The combined organic layers were washed with water (3 x 30 mL), dried over anhydrous Na₂SO₄. After filtration, the filtrate was concentrated under reduced pressure. The crude product was purified by Prep-HPLC with the following conditions (Column: Xselect CSH C18 OBD Column 30*150mm 5µm, n; Mobile Phase A: Water(0.05%TFA ), Mobile Phase B: MeOH-HPLC; Flow rate: 60 mL/min; Gradient: 3% B to 33% B in 10 min, 33% B; Wave Length: 254 nm; RT1(min): 8.55; Number Of Runs: 4) to afford 1-(2,6-dimethylmorpholino)-3-(3-(3-hydroxy-4-methoxyphenyl)-1H-pyrazol-1-yl)propan-1-one (28.4 mg, 33.79%) as a white solid. ¹HNMR (300 MHz, DMSO-d₆) δ 9.02 (s, 1H), 7.77 (d, J = 2.4 Hz, 1H), 7.22 (d, J = 2.0 Hz, 1H), 7.16-7.11 (m, 1H), 6.92-6.88 (m, 1H), 6.57-6.55 (m, 1H), 5.64-5.56 (m, 1H), 4.23-4.19 (m, 1H), 4.04-3.96 (m, 1H), 3.76 (s, 3H), 3.55-3.40 (m, 1H), 3.31-2.94 (m, 1H), 2.76-2.58 (m, 1H), 2.34-2.23 (m, 1H), 1.57-1.52 (m, 3H), 1.09-0.93 (m, 6H). LC/MS: 360 [M+H]⁺.

### RICE03

### General Reaction scheme

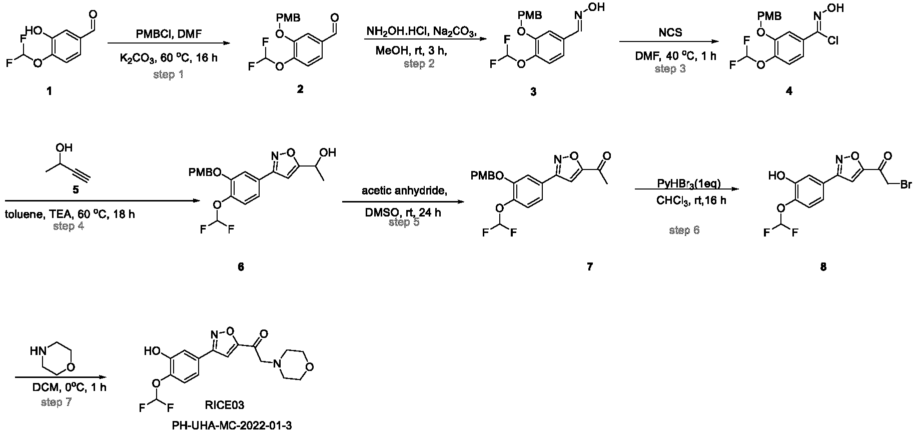

### Synthesis of 4-(difluoromethoxy)-3-((4-methoxybenzyl)oxy)benzaldehyde

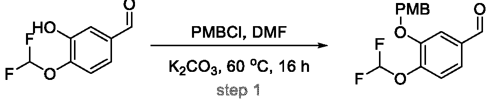

To a stirred solution of 4-(difluoromethoxy)-3-hydroxybenzaldehyde (10 g, 53.155 mmol, 1 equiv) and K₂CO₃ (9.55 g, 69.102 mmol, 1.3 equiv) in DMF (100 mL) were added PMBCl (9.16 g, 58.471 mmol, 1.1 equiv) dropwise at 25 °C. The resulting mixture was stirred for 16 h at 60 °C. The resulting mixture was filtered, the filter cake was washed with EtOAc (3x100 mL). The filtrate was concentrated under reduced pressure. The residue was purified by silica gel column chromatography, eluted with PE/EA (1:1) to afford 4-(difluoromethoxy)-3-[(4-methoxyphenyl)methoxy]benzaldehyde (16 g, 87.88%) as a white solid. LC/MS: 309 [M+H]⁺

### Synthesis of 4-(difluoromethoxy)-3-((4-methoxybenzyl)oxy)benzaldehyde oxime

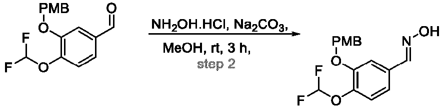

To a stirred solution of 4-(difluoromethoxy)-3-[(4-methoxyphenyl)methoxy]benzaldehyde (14 g, 45.413 mmol, 1 equiv) and Na₂CO₃ (2.89 g, 27.267 mmol, 0.60 equiv) in MeOH (280 mL) were added NH₂OH.HCl (3.79 g, 54.496 mmol, 1.2 equiv) in portions at room temperature. The resulting mixture was stirred for 3 h at room temperature. The resulting mixture was concentrated under reduced pressure. The resulting mixture was diluted with water (300 mL). The resulting mixture was extracted with EtOAc (3 x 200 mL). The combined organic layers were washed with brine (3x100 mL), dried over anhydrous Na₂SO₄. After filtration, the filtrate was concentrated under reduced pressure. This resulted in 4-(difluoromethoxy)-3-((4-methoxybenzyl)oxy)benzaldehyde oxime (14 g, 85.82%) as a white solid. LC/MS: 324 [M+H]⁺

### Synthesis of 4-(difluoromethoxy)-N-hydroxy-3-((4-methoxybenzyl)oxy)benzimidoyl chloride

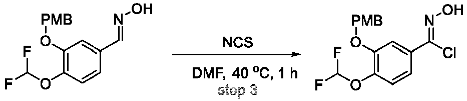

To a stirred solution of 4-(difluoromethoxy)-3-((4-methoxybenzyl)oxy)benzaldehyde oxime (12 g, 37.118 mmol, 1 equiv) in DMF (120 mL) was added NCS (5.20 g, 38.974 mmol, 1.05 equiv) in portions at room temperature. The resulting mixture was stirred for 1 h at 40 °C. The resulting mixture was diluted with water (300 mL). The resulting mixture was extracted with EtOAc (3 x 300 mL). The combined organic layers were washed with brine (3x300 mL), dried over anhydrous Na₂SO₄. After filtration, the filtrate was concentrated under reduced pressure. This resulted in 4-(difluoromethoxy)-N-hydroxy-3-((4-methoxybenzyl)oxy)benzimidoyl chloride (12 g, 90.37%) as a yellow oil. LC/MS: 358 [M+H]⁺

### 4. Synthesis of 1-(3-(4-(difluoromethoxy)-3-((4-methoxybenzyl)oxy)phenyl)isoxazole-5-yl)ethan-1-ol

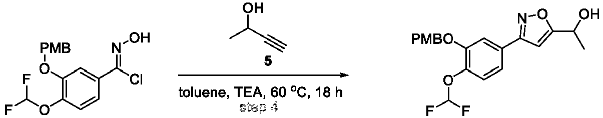

To a stirred solution of 4-(difluoromethoxy)-N-hydroxy-3-((4-methoxybenzyl)oxy)benzimidoyl chloride (12 g, 33.544 mmol, 1 equiv) in Toluene (200 mL) were added 3-butyn-2-ol (2.35 g, 33.544 mmol, 1 equiv) and TEA (5.09 g, 50.316 mmol, 1.5 equiv) dropwise at 0 °C. The resulting mixture was stirred for16 h at 60 °C. The mixture was allowed to cool down to room temperature. The resulting mixture was diluted with water (300 mL). The resulting mixture was extracted with EtOAc (3 x 300 mL). The combined organic layers were washed with brine (300 mL), dried over anhydrous Na₂SO₄. After filtration, the filtrate was concentrated under reduced pressure. The residue was purified by silica gel column chromatography, eluted with PE/EA (1:1) to afford 1-(3-(4-(difluoromethoxy)-3-((4-methoxybenzyl)oxy)phenyl)isoxazole-5-yl)ethan-1-ol (7.5 g, 51.42%) as a yellow solid. LC/MS: 392 [M+H]⁺

### 5. Synthesis of 1-(3-(4-(difluoromethoxy)-3-((4-methoxybenzyl)oxy)phenyl)isoxazole-5-yl)ethan-1-one

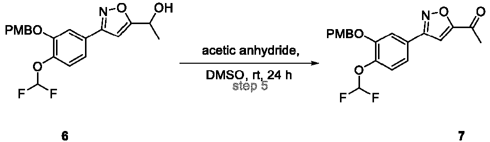

To a stirred solution of 1-(3-(4-(difluoromethoxy)-3-((4-methoxybenzyl)oxy)phenyl)isoxazole-5-yl)ethan-1-ol (7.5 g, 19.163 mmol, 1 equiv) in DMSO (50 mL) was added Ac₂O (15 mL) at room temperature. The resulting mixture was stirred for 16 h at room temperature. The resulting mixture was diluted with water (200 mL). The resulting mixture was extracted with EtOAc (3 x 200 mL). The combined organic layers were washed with brine (3x300 mL), dried over anhydrous Na₂SO₄. After filtration, the filtrate was concentrated under reduced pressure. The residue was purified by silica gel column chromatography, eluted with PE / EA (2:1) to afford 1-(3-(4-(difluoromethoxy)-3-((4-methoxybenzyl)oxy)phenyl)isoxazole-5-yl)ethan-1-one (5.5 g, 66.34%) as a yellow solid. LC/MS: 390 [M+H]⁺

### 6. Synthesis of 2-bromo-1-(3-(4-(difluoromethoxy)-3-hydroxyphenyl)isoxazole-5-yl)ethan-1-one

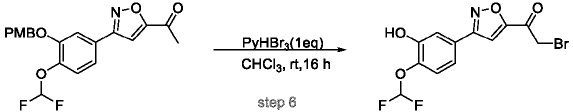

To a stirred solution of 1-(3-(4-(difluoromethoxy)-3-((4-methoxybenzyl)oxy)phenyl)isoxazole-5-yl)ethan-1-one (25 mg, 0.064 mmol, 1 equiv) in CHCl₃ (2 mL) was added Pyridinium tribromide (20.55 mg, 0.064 mmol, 1 equiv) at room temperature. The reaction was stirred 20 batches in parallels. The reaction was quenched by the addition of sat. sodium bisulfite (aq.) (100 mL) at room temperature. The resulting mixture was extracted with EtOAc (3 x100 mL). The combined organic layers were washed with bicarbonate (100 mL), dried over anhydrous Na₂SO₄. After filtration, the filtrate was concentrated under reduced pressure. This resulted in 2-bromo-1-(3-(4-(difluoromethoxy)-3-hydroxyphenyl)isoxazole-5-yl)ethan-1-one (500 mg, crude) as a yellow oil. LC/MS: 348 [M+H]⁺

### 7. Synthesis of 1-(3-(4-(difluoromethoxy)-3-hydroxyphenyl)isoxazole-5-yl)-2-morpholinoethan-1-one

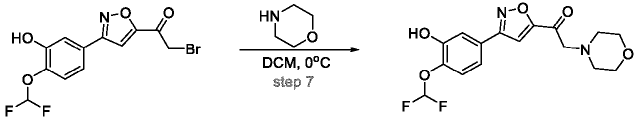

To a stirred solution of 2-bromo-1-(3-(4-(difluoromethoxy)-3-hydroxyphenyl)isoxazole-5-yl)ethan-1-one (500 mg, 1.436 mmol, 1 equiv) in DCM(20 mL) was added morpholine (250.28 mg, 2.872 mmol, 2 equiv) dropwise at 0 °C. The resulting mixture was stirred for 1 h at 0 °C. The residue was dissolved in water (100 mL). The resulting mixture was extracted with CH₂Cl₂ (3 x 100 mL). The combined organic layers were washed with brine (3x100 mL), dried over anhydrous Na₂SO₄. After filtration, the filtrate was concentrated under reduced pressure. The residue was purified by silica gel column chromatography, eluted with PE / EA (1:1) to afford crude product. The crude product (50 mg) was purified by Prep-HPLC with the following conditions (Column: Xselect CSH Prep C18 OBD Column, 19*250 mm, 5 µ m; Mobile Phase A: Water (0.05% HCl), Mobile Phase B: CAN; Flow rate: 25 mL/min; Gradient: 5% B to 28% B in 10 min, 28% B; Wave Length: 254 nm; RT1(min): 9.85; Number Of Runs: 3) to afford 1-(3-(4-(difluoromethoxy)-3-hydroxyphenyl)isoxazole-5-yl)-2-morpholinoethan-1-one (1.6 mg, 0.29%) as a colorless oil. ¹HNMR (300 MHz, Methanol-*d*₄) δ 7.78(s, 1H), 7.66-7.53 (m, 1H), 7.40-7.23 (m, 2H), 6.88 (t, J = 76 Hz, 1H), 3.91-3.88 (m, 4H), 3.69-3.50 (m, 2H), 3.69- 3.56 (m, 2H), 3.26-3.22 (m, 1H), 1.22-1.17 (m, 1H). LC/MS: 355 [M+H]⁺.

### RICE04

### General Reaction Scheme

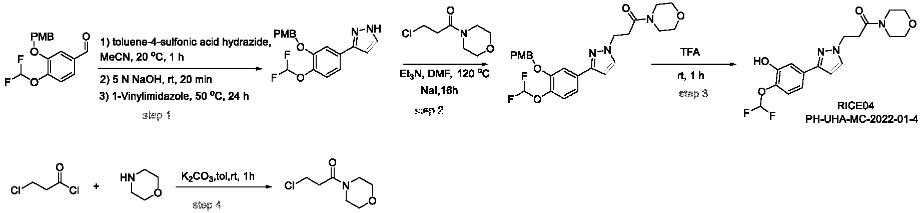

### Step 1. Synthesis of 3-[4-(Difluoromethoxy)-3-[(4-methoxyphenyl)methoxy]phenyl]-1H-pyrazole

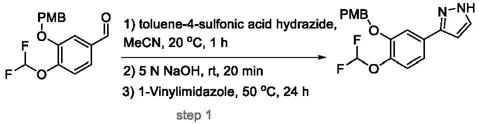

To a solution of 4-(difluoromethoxy)-3-[(4-methoxyphenyl)methoxy]benzaldehyde (15 g, 48.6 mmol, 1 equiv) in CAN (300 mL) was added 4-toluenesulfonyl hydrazide (9.97 g, 53.5 mmol, 1.1 equiv) in portions and the mixture was stirred for 1 h at room temperature. TLC showed the starting material was consumed. Then NaOH (10.7 mL, 53.5 mmol, 1.1 equiv) was added to the above mixture, and the mixture was stirred for 20 min at the same temperature. After that, 1-ethenylimidazole (22.9 g, 243.3 mmol, 5 equiv) was slowly added to the above mixture and the mixture was stirred for 16 h at 50 °C. After cooling to room temperature, the solvent was removed under reduced pressure and the residue was poured into water (200 mL). The mixture was extracted with EA (2 x 200 mL). The organic layers were concentrated under reduced pressure to give the crude product. The crude product was purified by silica gel column chromatography, eluted with petroleum ether / ethyl acetate (1:1) to afford 3-[4-(difluoromethoxy)-3-[(4-methoxyphenyl)methoxy]phenyl]-1H-pyrazole (12 g, 64.1%) as a white solid. LC/MS: 347 [M+H]⁺.

### Step 2a. Synthesis of 3-chloro-1-morpholinopropan-1-one

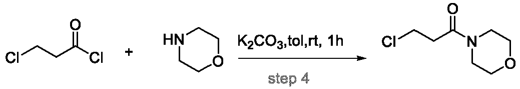

To a stirred mixture of morpholine (5 g, 57.3 mmol, 1 equiv) and K₂CO₃ (15.8 g, 114 mmol, 2 equiv) in toluene (60 mL) was added 3-chloro-propanoyl chloride (7.29 g, 57.3 mmol, 1 equiv) at 0 °C under nitrogen atmosphere. The resulting mixture was stirred for1 h at room temperature. The resulting mixture was poured into water (200 mL) and extracted with EtOAc (3 x 300 mL). The combined organic layers were washed with brine (2 x 200 mL), dried over anhydrous Na₂SO₄. After filtration, the filtrate was concentrated under reduced pressure. This is to afford 3-chloro-1-(morpholin-4-yl)propan-1-one (4.7 g, 46.1%) as a white solid. The crude product was used in the next step directly without further purification. LC-MS: m/z 178 [M+H]⁺.

### Step 2b. Synthesis of 3-(3-(4-(difluoromethoxy)-3-((4-methoxybenzyl)oxy)phenyl)-1H-pyrazol-1-yl)-1-morpholinopropan-1-one

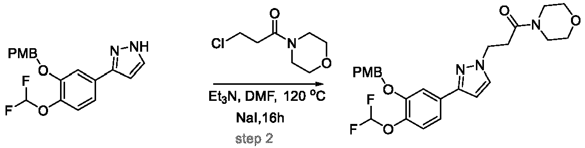

To a stirred mixture of 3-(4-(difluoromethoxy)-3-((4-methoxybenzyl)oxy)phenyl)-1H-pyrazole (600 mg, 1.73 mmol, 1 equiv) and 3-chloro-1-(morpholin-4-yl)propan-1-one (1.85 g, 10.3 mmol, 6 equiv) in DMF(10 mL) were added triethylamine (525 mg, 5.19 mmol, 3 equiv) and NaI (129 mg, 0.866 mmol, 0.5 equiv). The resulting mixture was stirred for 24 h at 120 °C under nitrogen atmosphere. The mixture was allowed to cool down to room temperature, poured into water (100 mL) and extracted with EtOAc (3 x 100 mL). The combined organic layers were washed with brine (3 x 150 mL), dried over anhydrous Na₂SO₄. After filtration, the filtrate was concentrated under reduced pressure. The residue was purified by Prep-TLC (CH₂Cl₂ / MeOH=25:1) to afford 3-(3-(4-(difluoromethoxy)-3-((4-methoxybenzyl)oxy)phenyl)-1H-pyrazol-1-yl)-1-morpholinopropan-1-one (340 mg, 40.2%) as a yellow oil. LC/MS: 488 [M+H]⁺.

### Step 3. Synthesis of 3-(3-(4-(difluoromethoxy)-3-hydroxyphenyl)-1H-pyrazol-1-yl)-1-morpholinopropan-1-one

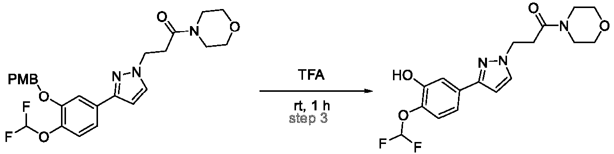

A solution of 3-(3-(4-(difluoromethoxy)-3-((4-methoxybenzyl)oxy)phenyl)-1H-pyrazol-1-yl)-1-morpholinopropan-1-one (340 mg, 0.697 mmol, 1 equiv) in TFA (4 mL) was stirred for 1 h at room temperature. The solution was concentrated under vacuum. The crude product was purified by Prep-HPLC with the following conditions (Column: Xbridge Prep OBD C18 Column, 30 x 150 mm, 5µm; Mobile Phase A: Water(10 mmol/L NH₄HCO₃+0.1%NH₃.H₂O), Mobile Phase B: CAN; Flow rate: 60 mL/min; Gradient: 17% B to 47% B in 7 min, 47% B; Wave Length: 254 nm; RT1(min): 5.20) .The product fractions were lyophilized to afford 3-(3-(4-(difluoromethoxy)-3-hydroxyphenyl)-1H-pyrazol-1-yl)-1-morpholinopropan-1-one (33.5 mg, 12.4%) as a white solid.¹H NMR (400 MHz, DMSO-d₆) δ 9.98 (br s, 1H), 7.75 (d, *J* = 2.0 Hz, 1H), 7.40 (d, *J =* 2.0 Hz, 1H), 7.23-6.85 (m, 3H), 6.56-6.55 (m, 1H), 4.38-4.32 (m, 2H), 3.73-3.34 (m, 8H), 2.93-2.82(m, 2H) LC-MS: m/z 368 [M+H]⁺.

### RICE05

### General synthesis scheme

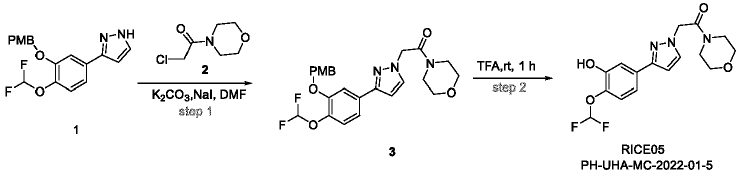

### Step 1. Synthesis of 2-{3-[4-(difluoromethoxy)-3-[(4-methoxyphenyl)methoxy]phenyl]pyrazol-1-yl}-1-(morpholin-4-yl)isoxazol

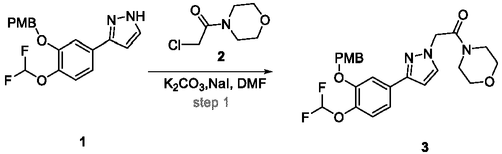

To a stirred solution of 3-[4-(difluoromethoxy)-3-[(4-methoxyphenyl)methoxy]phenyl]-1H-pyrazole (500 mg, 1.44 mmol, 1 equiv) and 2-chloro-1-morpholinoethan-1-one (472 mg, 2.88 mmol, 2 equiv) in DMF (10 mL) were added K₂CO₃ (399 mg, 2.88 mmol, 2 equiv) and NaI (108 mg, 0.722 mmol, 0.5 equiv). The resulting mixture was stirred for 5 h at 60 °C. The mixture was allowed to cool down to room temperature, poured into water (100 mL) and extracted with EtOAc (2 x 100 mL). The combined organic layers were concentrated under reduced pressure. The residue was purified by silica gel column chromatography, eluted with PE / EA (1:1) to afford 2-{3-[4-(difluoromethoxy)-3-[(4-methoxyphenyl)methoxy]phenyl]pyrazol-1-yl}-1-(morpholin-4-yl)isoxazol (500 mg, 73.1%) as an off-white solid. LC/MS: 474 [M+H]⁺.

### Step 2. Synthesis of 3-(3-(4-(difluoromethoxy)-3-hydroxyphenyl)-1H-pyrazol-1-yl)-1-morpholinopropan-1-one

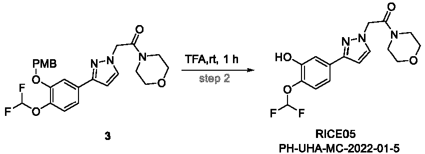

A solution of 2-{3-[4-(difluoromethoxy)-3-[(4-methoxyphenyl)methoxy]phenyl]pyrazol-1-yl}-1-(morpholin-4-yl)isoxazol (100 mg, 0.211 mmol, 1 equiv) in TFA (2 mL) was stirred for 1 h at 25 °C. The resulting mixture was concentrated under vacuum. The residue was purified by Prep-HPLC with the following conditions (Column: Xbridge Prep OBD C18 Column, 30 x 150 mm, 5µm; Mobile Phase A: Water (10 mmol/L NH₄HCO₃+0.1%NH₃.H₂O), Mobile Phase B: CAN; Flow rate: 60 mL/min; Gradient: 17% B to 47% B in 7 min, 47% B; Wave Length: 254 nm; RT1(min): 5.20). The product fractions were lyophilized to afford 2-{3-[4-(difluoromethoxy)-3-hydroxyphenyl]pyrazol-1-yl}-1-(morpholin-4-yl)isoxazol (30.5 mg, 40.7%) as a white solid. ¹H NMR (400 MHz, DMSO-d₆) δ 9.97 (br s, 1H), 7.69 (d, *J =* 2.4 Hz, 1H), 7.39 (d, *J* = 2.0 Hz, 1H), 7.24-7.18 (m, 1H), 7.11-7.09 (m, 1H), 7.24-6.86 (m, 1H), 6.62 (d, *J* = 2.4 Hz, 1H), 5.17 (s, 2H), 3.64-3.60 (m, 4H), 3.59-3.57 (m, 2H), 3.47-3.33 (m, 2H). LC-MS: m/z 354 [M+H]⁺.

### RICE06

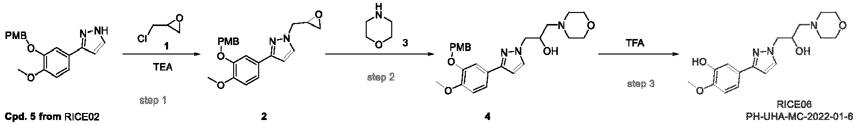

### 3-(4-methoxy-3-((4-methoxybenzyl)oxy)phenyl)-1-(oxiran-2-ylmethyl)-1H-pyrazole

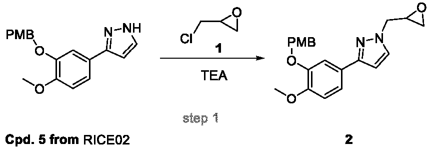

To a mixture of 3-(4-methoxy-3-((4-methoxybenzyl)oxy)phenyl)-1H-pyrazole (500 mg, 1.611 mmol, 1 equiv) and epichlorohydrin (3 mL) was added TEA (244 mg, 2.4 mmol, 1.5 equiv) dropwise at 0 °C. The resulting mixture was stirred for 3 h at 70°C. After cooling to room temperature, the reaction mixture was quenched with water (50 mL) The mixture was extracted with EtOAc (3 x 50mL). The combined organic layers were washed with water (3 x 50 mL), dried over anhydrous Na₂SO₄. After filtration, the filtrate was concentrated under reduced pressure. The residue was purified by silica gel column chromatography, eluted with EtOAc/PE (1:1) to afford 3-(4-methoxy-3-((4-methoxybenzyl)oxy)phenyl)-1-(oxiran-2-ylmethyl)-1H-pyrazole (260 mg, 25.55%) as a light yellow oil. LC/MS: 367 [M+H]⁺

### Synthesis of 1-(3-(4-methoxy-3-((4-methoxybenzyl)oxy)phenyl)-1H-pyrazol-1-yl)-3-morpholinopropan-2-ol

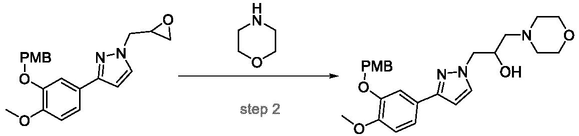

A solution of 3-(4-methoxy-3-((4-methoxybenzyl)oxy)phenyl)-1-(oxiran-2-ylmethyl)-1H-pyrazole (260 mg, 0.710 mmol, 1 equiv) in morpholine (5 mL) was stirred for 24 h at 60 °C. The resulting mixture was extracted with EtOAc (3 x 50mL). The combined organic layers were washed with water (3 x 50 mL), dried over anhydrous Na₂SO₄. After filtration, the filtrate was concentrated under reduced pressure. The residue was purified by silica gel column chromatography, eluted with DCM/MeOH (10:1) to afford 1-(3-(4-methoxy-3-((4-methoxybenzyl)oxy)phenyl)-1H-pyrazol-1-yl)-3-morpholinopropan-2-ol (190 mg, 34.07%) as a light yellow oil. LC/MS: 454 [M+H]⁺

### Synthesis of 5-(1-(2-hydroxy-3-morpholinopropyl)-1H-pyrazol-3-yl)-2-methoxyphenol

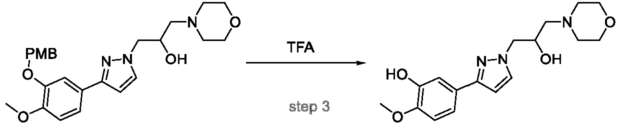

To a solution of 1-(3-{4-methoxy-3-[(4-methoxyphenyl)methoxy]phenyl}pyrazol-1-yl)-3-(morpholin-4-yl)propan-2-ol (190 mg, 0.251 mmol, 1 equiv) was added TFA (2 mL) . The mixture was stirred at 25 °C FOR 1 h. The reaction was quenched with water (3 x 50 mL). The resulting mixture was extracted with EtOAc (3 x 50 mL). The combined organic layers were washed with water (3 x 50 mL), dried over anhydrous Na₂SO₄. After filtration, the filtrate was concentrated under reduced pressure. The crude product was purified by Prep-HPLC with the following conditions (Column: Xselect CSH C18 OBD Column 30*150mm 5µm, n; Mobile Phase A: Water(0.05%TFA), Mobile Phase B: MeOH-HPLC; Flow rate: 60 mL/min; Gradient: 3% B to 33% B in 10 min, 33% B; Wave Length: 254 nm; RT1(min): 8.55/9.10; Number Of Runs: 3) to afford 5-(1-(2-hydroxy-3-morpholinopropyl)-1H-pyrazol-3-yl)-2-methoxyphenol (28.4 mg, 33.79%) as a white solid.
¹HNMR (300 MHz, DMSO-d₆+D₂O) δ 7.70 (d, J = 2.4 Hz, 1H), 7.19-7.14 (m, 2H), 6.90 (d, J = 8.4, 1H), 6.54 (d, J = 2.4 Hz, 1H), 4.35-4.25 (m, 1H), 4.21- 4.06 (m, 2H), 3.90- 3.85 (m, 2H), 3.80 (s, 3H), 3.75 - 3.70 (m, 2H), 3.34-3.30 (m, 2H), 3.25-3.03(m, 4H). LC/MS: 334 [M+H]⁺.

### RICE07

### General synthesis scheme

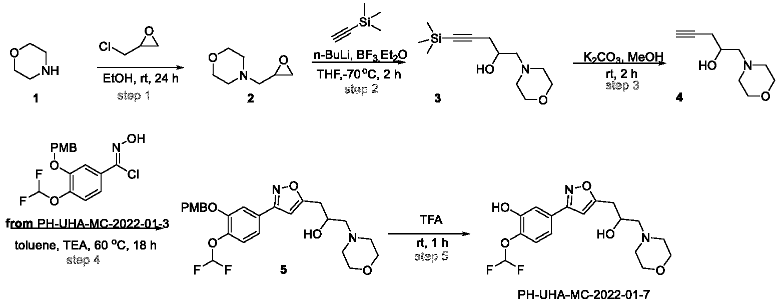

### Synthesis of 4-(oxiran-2-ylmethyl)morpholine

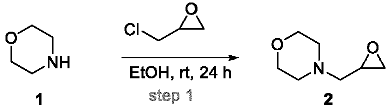

A solution of morpholine (5 g, 57.391 mmol, 1 equiv) and epichlorohydrin (5.31 g, 57.391 mmol, 1 equiv) in EtOH (150 mL) was stirred for 24 h at room temperature. The resulting mixture was concentrated under reduced pressure. This resulted in 4-(oxiran-2-ylmethyl)morpholine (9 g, crude) as a colorless oil. The crude product mixture was used in the next step directly without further purification. LC/MS: 144.09 [M+H]⁺

### Synthesis of 1-morpholino-5-(trimethylsilyl)pent-4-yn-2-ol

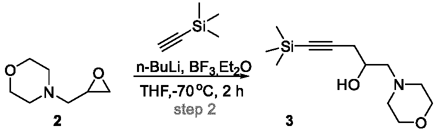

A solution of trimethylsilylacetylene (1.5 g, 15.272 mmol, 1.82 equiv) in THF (50 mL) was treated with n-BuLi in hexanes (6 mL, 15.000 mmol, 1.79 equiv) dropwise for 30 min at -70 °C under nitrogen atmosphere followed by the addition of 4-(oxiran-2-ylmethyl)morpholine (2 g, 8.381 mmol, 1 equiv, 60%) in THF(5 mL) dropwise at -65°C. To the above mixture was added BF3.Et2O (1.19 g, 8.381 mmol, 1 equiv) in THF(5mL) dropwise. The resulting mixture was stirred for additional 1.5h at -70°C. The reaction was quenched with saturated NH₄Cl solution. The resulting mixture was extracted with EtOAc (3 x 50 mL). The combined organic layers were dried over anhydrous MgSO4. After filtration, the filtrate was concentrated under reduced pressure. The residue was purified by silica gel column chromatography, eluted with PE / EA (1:1) to afford 1-(morpholin-4-yl)-5-(trimethylsilyl)pent-4-yn-2-ol (500 mg, 24.71%) as a yellow oil. LC/MS: 242.15 [M+H]⁺

### Synthesis of 1-morpholinopent-4-yn-2-ol

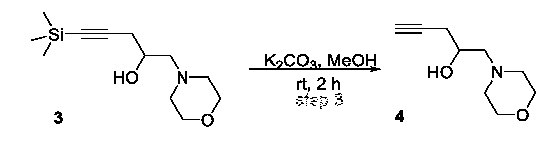

To a stirred solution of 1-(morpholin-4-yl)-5-(trimethylsilyl)pent-4-yn-2-ol (450 mg, 1.864 mmol, 1 equiv) in MeOH (20 mL) was added K2CO3 (772.88 mg, 5.592 mmol, 3 equiv) in portions at 25°C. The resulting mixture was stirred for 2 h at 25°C. The resulting mixture was concentrated under reduced pressure. The residue was diluted with ethyl acetate and the resulting mixture was filtered; the filter cake was washed with ethyl acetate (2x20 mL). The filtrate was concentrated under reduced pressure. The crude product was used in the next step directly without further purification. This resulted in 1-(morpholin-4-yl)pent-4-yn-2-ol (300 mg, 95.10%) as a yellow oil. LC/MS: 170.11 [M+H]⁺.

### 4. Synthesis of 1-(3-(4-(difluoromethoxy)-3-((4-methoxybenzyl)oxy)phenyl)isoxazole-5-yl)-3-morpholinopropan-2-ol

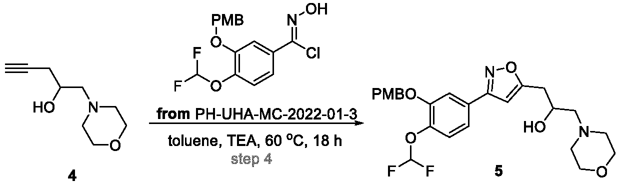

A solution of 1-(morpholin-4-yl)pent-4-yn-2-ol (500 mg, 2.955 mmol, 1 equiv) in Toluene (20 mL) was treated with (Z)-4-(difluoromethoxy)-N-hydroxy-3-[(4-methoxyphenyl)methoxy]benzenecarbonimidoyl chloride (1585.50 mg, 4.433 mmol, 1.5 equiv). The resulting mixture was stirred for overnight at 60°C. The reaction was quenched with Water(20mL). The resulting mixture was extracted with EtOAc (3 x 20 mL). The combined organic layers were dried over anhydrous Na2SO4. After filtration, the filtrate was concentrated under reduced pressure. The residue was purified by reverse flash chromatography with the following conditions: column, C18 silica gel; mobile phase, MeCN in Water (0.1% TFA), 10% to 100% gradient in 30 min; detector, UV 254 nm. The fraction was concentrated under vacuum to afford 1-{3-[4-(difluoromethoxy)-3-[(4-methoxyphenyl)methoxy]phenyl]-1,2-oxazol-5-yl}-3-(morpholin-4-yl)propan-2-ol (150 mg, 10.35%) as a yellow solid. LC/MS: 491.19 [M+H]⁺.

### 5. Synthesis of 2-(difluoromethoxy)-5-(5-(2-hydroxy-3-morpholinopropyl)isoxazole-3-yl)phenol

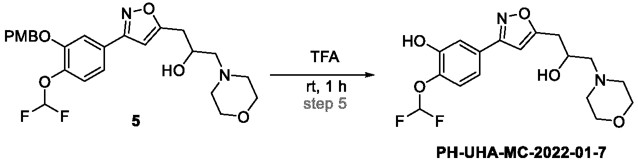

A solution of 2-(difluoromethoxy)-5-{5-[2-hydroxy-3-(morpholin-4-yl)propyl]-1,2-oxazol-3-yl}phenol (200 mg, 0.540 mmol, 1 equiv) in TFA (5 mL, 0.044 mmol, 0.14 equiv) was stirred for 1 h at room temperature. The resulting mixture was concentrated under reduced pressure. The crude product (110 mg) was purified by Prep-HPLC with the following conditions (Column: Xbridge Shield RP18 OBD Column, 30*150 mm, 5µm; Mobile Phase A: Water(10 mmol/L NH4HCO3), Mobile Phase B: CAN; Flow rate: 60 mL/min; Gradient: 17% B to 47% B in 7 min, 47% B; Wave Length: 254 nm; RT1(min): 4.67; Number Of Runs: 0) to afford 1-{3-[4-(difluoromethoxy)-3-[(4-methoxyphenyl)methoxy]phenyl]-1,2-oxazol-5-yl}-3-(morpholin-4-yl)propan-2-ol (31.8 mg, 11.97%) as an off-white solid. ¹HNMR (400 MHz, DMSO-d₆) δ 7.41 (s, 1H), 6.96-7.34 (m, 3H), 6.70 (s, 1H), 6.05 (br, 1H), 4.89(br, 1H), 4.00-4.03 (m, 1H), 3.55-3.57 (m, 4H), 2.97-3.06 (m, 1H), 2.75-2.86(m, 1H), 2.39 - 2.44 (m, 4H), 2.27-2.38 (m, 2H). ¹⁹FNMR (400 MHz, DMSO) δ -81.287. LC/MS: 371.10 [M+H]⁺.

### RICE08

### General synthesis scheme

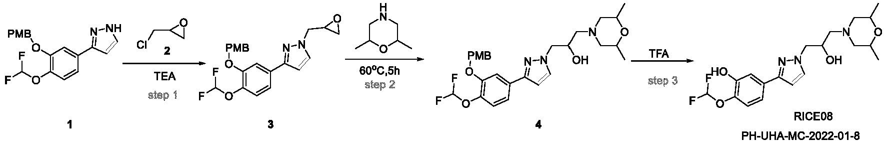

### Step 1. Synthesis of 3-[4-(difluoromethoxy)-3-[(4-methoxyphenyl)methoxy]phenyl]-1-(oxiran-2-ylmethyl)pyrazole

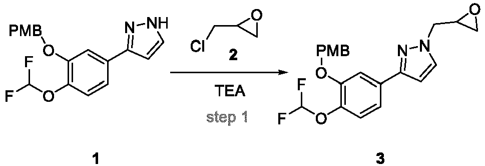

To a stirred solution of 3-[4-(difluoromethoxy)-3-[(4-methoxyphenyl)methoxy]phenyl]-1H-pyrazole (1 g, 2.9 mmol, 1 equiv) and epichlorohydrin (3.47 g, 37.5 mmol, 13 equiv) was added TEA (0.44 g, 4.33 mmol, 1.5 equiv). The resulting mixture was stirred for 3 h at 70 °C. The mixture was allowed to cool down to room temperature, poured into water (50 mL) and extracted with EtOAc (2 x 50 mL). The combined organic layers were concentrated under reduced pressure. The residue was purified by silica gel column chromatography, eluted with PE / EA (1:1) to afford 3-[4-(difluoromethoxy)-3-[(4-methoxyphenyl)methoxy]phenyl]-1-(oxiran-2-ylmethyl)pyrazole (800 mg, 68.8%) as a yellow oil. LC-MS: m/z 403 [M+H]⁺

### Step 2. Synthesis of 1-{3-[4-(difluoromethoxy)-3-[(4-methoxyphenyl)methoxy]phenyl]pyrazol-1-yl}-3-(2,6-dimethylmorpholin-4-yl)propan-2-ol

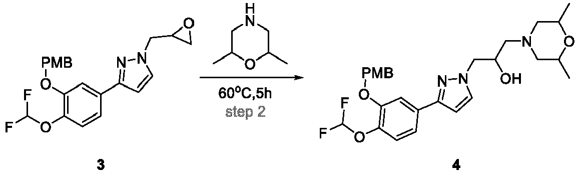

A solution of 3-[4-(difluoromethoxy)-3-[(4-methoxyphenyl)methoxy]phenyl]-1-(oxiran-2-ylmethyl)pyrazole (500 mg, 1.24 mmol, 1 equiv) in 2,6-dimethyl-morpholine (5 mL) was stirred for 5 h at 60 °C. The mixture was allowed to cool down to room temperature. The resulting mixture was poured into water (30 mL) and extracted with EtOAc (2 x 30 mL). The combined organic layers were concentrated under reduced pressure. The residue was purified by silica gel column chromatography, eluted with PE: EA (1:1) to afford 1-{3-[4-(difluoromethoxy)-3-[(4-methoxyphenyl)methoxy]phenyl]pyrazol-1-yl}-3-(2,6-dimethylmorpholin-4-yl)propan-2-ol (400 mg, 62.2%) as a yellow oil. The mixture was re-separated by prep-Achiral SFC with the following conditions: Column: YMC-Actus Triart Diol-HILIC, 3 x 25 cm, 5 µm; Flow rate: 65 mL/min; Gradient: isocratic 15% B; Column Temperature (°C): 35; Back Pressure (bar): 100; Wave Length: 254 nm; RT1(min): 8.03; RT2(min): 9.75; Injection Volume: 1.5 mL; Number Of Runs: 9. The product fractions were concentrated under vacuum to afford 1-{3-[4-(difluoromethoxy)-3-[(4-methoxyphenyl)methoxy]phenyl]pyrazol-1-yl}-3-(2,6-dimethylmorpholin-4-yl)propan-2-ol (400 mg, 62.2%) as a yellow oil. LC-MS: m/z 518 [M+H]⁺

### Step 3. Synthesis of 2-(difluoromethoxy)-5-{1-[3-(2,6-dimethylmorpholin-4-yl)-2-hydroxypropyl]pyrazol-3-yl}phenol

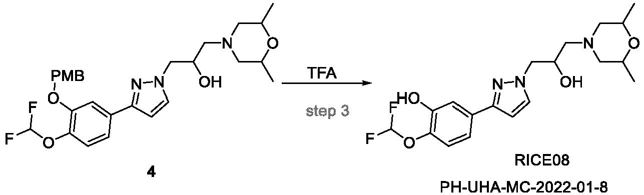

A solution of 1-{3-[4-(difluoromethoxy)-3-[(4-methoxyphenyl)methoxy]phenyl]pyrazol-1-yl}-3-(2,6-dimethylmorpholin-4-yl)propan-2-ol (300 mg, 0.58 mmol, 1 equiv) in TFA (3 mL) was stirred for 2 h at room temperature. The reaction mixture was concentrated under vacuum. The residue was purified by Prep-HPLC with the following conditions (Column: Xbridge Shield RP18 OBD Column, 30 x 150 mm, 5µm; Mobile Phase A: Water (10 mmol/L NH4HCO3), Mobile Phase B: CAN; Flow rate: 60 mL/min; Gradient: 22% B to 52% B in 7 min, 52% B; Wave Length: 254 nm; RT1(min): 5.02). The product fractions were lyophilized to afford 2-(difluoromethoxy)-5-{1-[3-(2,6-dimethylmorpholin-4-yl)-2-hydroxypropyl]pyrazol-3-yl}phenol (40.1 mg, 17.3%) as a white solid. ¹HNMR (300 MHz, Methanol-*d*₄) δ 7.64 (d, *J* = 2.1 Hz, 1H), 7.34 (d, *J* = 2.1 Hz, 1H), 7.24-7.21 (m, 1H), 7.16-7.01 (m, 1H), 6.76 (t, *J =* 75.3 Hz, 1H), 6.55 (d, *J =* 2.4 Hz, 1H), 4.33-4.27 (m, 1H), 4.22-4.06 (m, 2H), 3.74-3.65 (m, 2H), 2.82 (d, *J =* 12 Hz, 2H), 2.36 (d, *J =* 7.2 Hz, 2H), 1.83-1.71 (m, 2H), 1.11 (d, *J =* 6.3 Hz, 6H). LC-MS: m/z: 398.15 [M+H]⁺.

## Claims

1. Compound according to formula (I), or a stereoisomer, tautomer, racemic, salt, hydrate, N-oxide form, or solvate thereof, 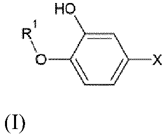 wherein:
R¹ = -CH₃ or -CHF₂;
X= 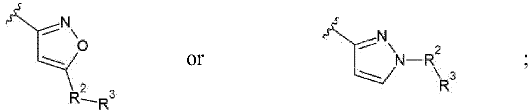
R² = -CO, -C=O(CH₂), -CH(OH)-CH₂, -CH₂-C=O, -CH₂-CH₂-C=O, -CH₂-CH(OH)-CH₂, -CH₂CH(OCOR⁴)-CH₂;
R³ = 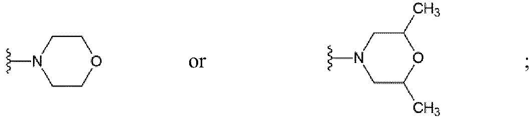 and
R⁴ = C₁-C₃ alkyl,

2. Compound according to claim 1, wherein R⁴ is -CH₃.

3. Compound according to any one of the preceding claims, wherein said compound is selected from the list comprising

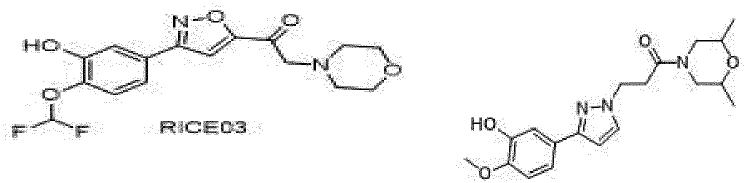

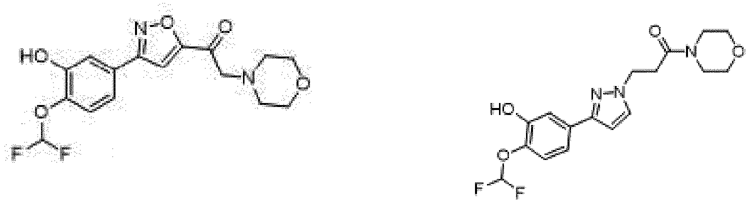

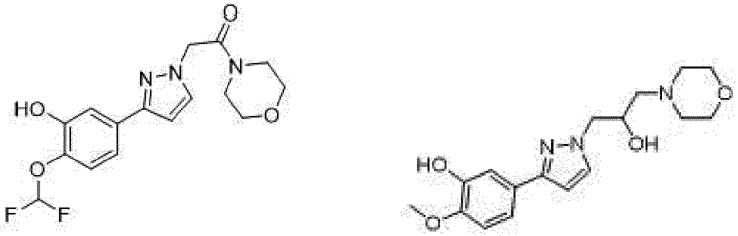

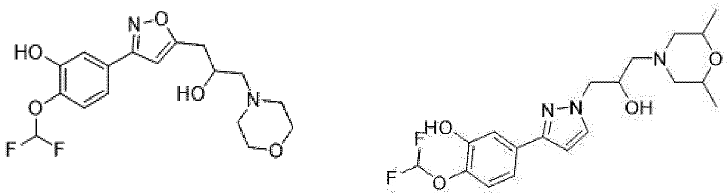

4. Pharmaceutical composition comprising at least one compound according to any one of the claims 1 to 3 and a pharmaceutical acceptable carrier.

5. A compound as defined in anyone of claims 1 to 3 or a pharmaceutical composition as defined in claim 4, for use in human or veterinary medicine.

6. A compound as defined in anyone of claims 1 to 3 or a pharmaceutical composition as defined in claim 4, for use as a medicament with PDE4D inhibiting activity.

7. A compound as defined in anyone of claims 1 to 3 or a pharmaceutical composition as defined in claim 4, for use in the prevention and/or treatment and/or cure of a neurodegenerative disorder in a subject.

8. The compound or pharmaceutical composition for use as defined in claim 7, wherein the neurodegenerative disorder is selected from the list comprising: Alzheimer's disease (AD), Parkinson's disease (PD), Huntington's disease, Lou Gehrig's disease (ALS), stroke, traumatic brain injury, spinal cord injury, mild cognitive impairment and demyelinating diseases such as peripheral demyelinating diseases, and multiple sclerosis (MS).

9. The compound or pharmaceutical composition for use as defined in claim 8, wherein the multiple sclerosis is progressive multiple sclerosis such as selected from the group comprising primary progressive multiple sclerosis, secondary progressive multiple sclerosis and relapse remitting multiple sclerosis.

10. A PDE4D inhibitor comprising a compound according to any one of the claims 1 to 3 or a pharmaceutical composition according to claim 4, wherein the PDE4D inhibitor selectively inhibits the type D isoforms of PDE4.

11. Method for synthesis of a compound according to any one of the claims 1 to 2, comprising the steps of:
- providing a compound according to formula (II); 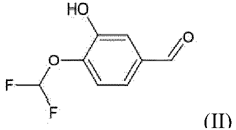
- reacting the compound according to formula (II) with 4-Methoxybenzyl chloride (PMBCl) forming a compound according to formula (III); 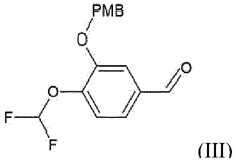
- dissolving the compound according formula (III) in acetonitrile (MeCN);
- reacting the dissolved compound according to formula (III) to form the compound according to formula (IV), 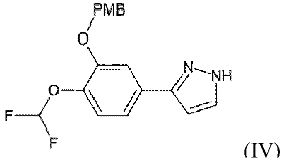 wherein the step of reacting comprises:
- adding toluene-4-sulfonic acid hydrazide to the dissolved compound according to formula (III) forming a reaction mixture;
- adding sodium hydroxide (NaOH) to the reaction mixture; and
- adding 1-vinlyimidazole to the reaction mixture;
- reacting the compound according formula (IV) with 2-(chloromethyl)oxirane and trimethylamine (Et₃N) to form the compound according to formula (V); 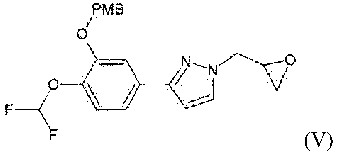
- reacting the compound according formula (V) with morpholine to form the compound according to formula (VI); and 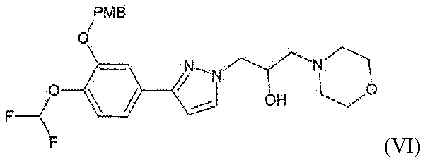
- reacting the compound according to formula (VI) with trifluoroacetic acid (TFA) to form the compound according to formula (I').

12. Method according to claim 11, wherein the step of adding toluene-4-sulfonic acid hydrazide to the dissolved compound according to formula (III) forming a reaction mixture is performed at a temperature in the range of 15 °C to 40 °C, preferably at a temperature in the range of 15 °C to 30 °C, more preferably at a temperature of about 20 °C.

13. Method according to claims 11 or 12, wherein the step of adding toluene-4-sulfonic acid hydrazide to the dissolved compound according to formula (III) forming a reaction mixture is performed for at least 45 minutes, more preferably performed for at least 55 minutes, more preferably performed for about 60 minutes.

14. Method according to anyone of claims 11-13, wherein the step of adding sodium hydroxide (NaOH) to the reaction mixture is performed for at least 10 minutes, preferably for at least 15 minutes, more preferably for at least 20 minutes.

15. Method according to any one of the claims 11-14, wherein the step of reacting the compound according formula (V) with morpholine to form the compound according to formula (VI) is performed at a temperature in the range of 30 °C to 80 °C, preferably performed at a temperature in the range of 40 °C to 70 °C, more preferably performed at a temperature in the range of 50 °C to 60 °C.

## Patentansprüche

1. Verbindung gemäß Formel (I) oder Stereoisomer, Tautomer, Racemat, Salz, Hydrat, N-Oxid-Form oder Solvat davon, 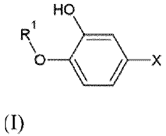 wobei:
R¹ = -CH₃ oder -CHF₂;
X= 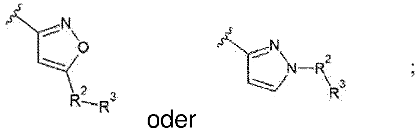
R² = -CO, -C=O(CH₂), -CH(OH)-CH₂, -CH₂-C=O, -CH₂-CH₂-C=O, -CH₂-CH(OH)-CH₂, -CH₂CH(OCOR⁴)-CH₂;
R³ = 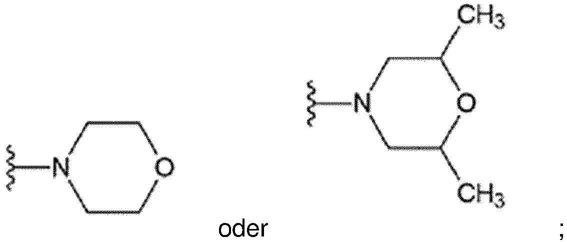 und
R⁴ = C₁-C₃-Alkyl.

2. Verbindung nach Anspruch 1, wobei R⁴ -CH₃ ist.

3. Verbindung nach einem der vorstehenden Ansprüche, wobei die Verbindung aus der Liste ausgewählt ist, umfassend

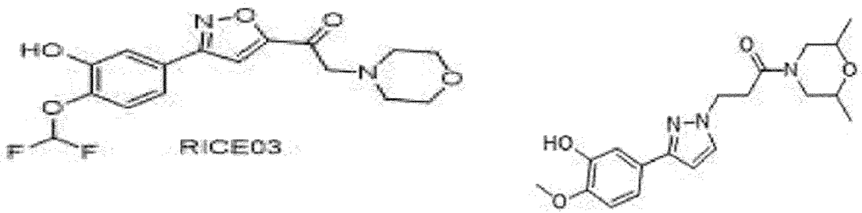

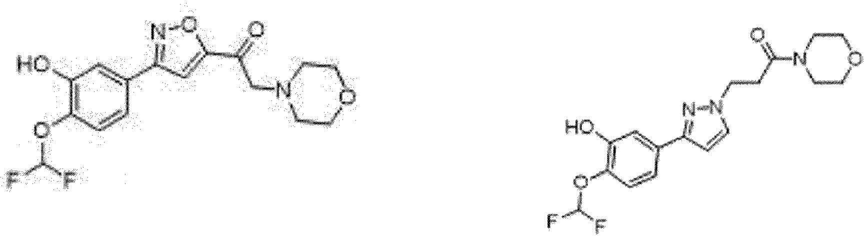

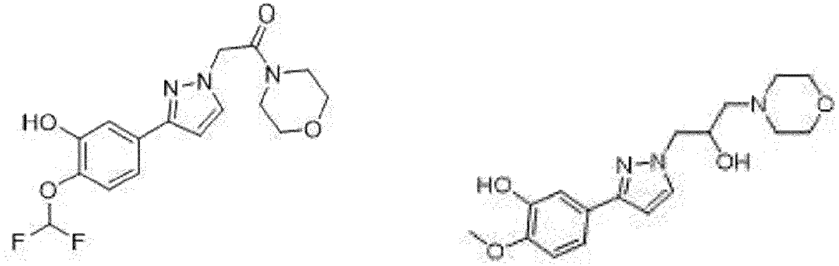

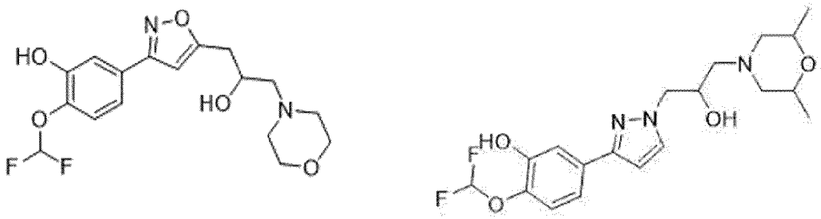

4. Pharmazeutische Zusammensetzung, umfassend mindestens eine Verbindung nach einem der Ansprüche 1 bis 3 und einen pharmazeutisch verträglichen Träger.

5. Verbindung, wie in einem der Ansprüche 1 bis 3 definiert, oder Zusammensetzung, wie in Anspruch 4 definiert, zur Verwendung in der Humanoder Veterinärmedizin.

6. Verbindung, wie in einem der Ansprüche 1 bis 3 definiert, oder Zusammensetzung, wie in Anspruch 4 definiert, zur Verwendung als ein Arzneimittel mit PDE4D-hemmender Aktivität.

7. Verbindung, wie in einem der Ansprüche 1 bis 3 definiert, oder Zusammensetzung, wie in Anspruch 4 definiert, zur Verwendung bei der Vorbeugung und/oder Behandlung und/oder Heilung einer neurodegenerativen Krankheit bei einem Subjekt.

8. Verbindung oder pharmazeutische Zusammensetzung zur Verwendung, wie in Anspruch 7 definiert, wobei die neurodegenerative Krankheit aus der Liste ausgewählt ist, umfassend: Alzheimer-Krankheit (AD), Parkinson-Krankheit (PD), Huntington-Krankheit, Lou-Gehrig-Krankheit (ALS), Schlaganfall, Schädel-Hirn-Verletzung, Rückenmarksverletzung, leichte kognitive Beeinträchtigung und demyelinisierende Krankheiten, wie periphere demyelinisierende Krankheiten, und multiple Sklerose (MS).

9. Verbindung oder pharmazeutische Zusammensetzung zur Verwendung, wie in Anspruch 8 definiert, wobei die multiple Sklerose progressive multiple Sklerose ist, wie aus der Gruppe ausgewählt, umfassend primäre progressive multiple Sklerose, sekundäre progressive multiple Sklerose und schubförmig remittierende multiple Sklerose.

10. PDE4D-Hemmstoff, umfassend eine Verbindung nach einem der Ansprüche 1 bis 3 oder eine pharmazeutische Zusammensetzung nach Anspruch 4, wobei der PDE4D-Hemmstoff die Typ-D-Isoformen von PDE4 selektiv hemmt.

11. Verfahren für eine Synthese einer Verbindung nach einem der Ansprüche 1 bis 2, umfassend die Schritte:
- Bereitstellen einer Verbindung gemäß Formel (II); 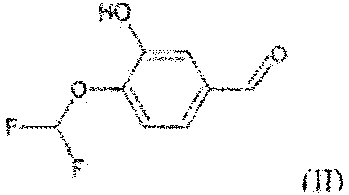
- Reagieren der Verbindung gemäß Formel (II) mit 4-Methoxybenzylchlorid (PMBC1), wobei eine Verbindung gemäß Formel (III) ausgebildet wird; 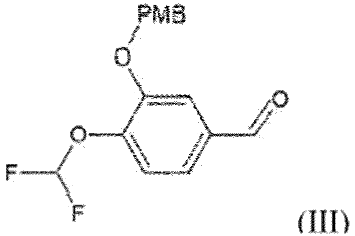
- Auflösen der Verbindung gemäß Formel (III) in Acetonitril (MeCN);
- Reagieren der gelösten Verbindung gemäß Formel (III), um die Verbindung gemäß Formel (IV) auszubilden, 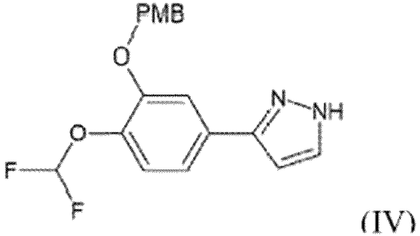 wobei der Schritt des Reagierens umfasst:
- Hinzufügen von Toluol-4-sulfonsäurehydrazid zu der gelösten Verbindung gemäß Formel (III), wobei ein Reaktionsgemisch ausgebildet wird;
- Hinzufügen von Natriumhydroxid (NaOH) zu dem Reaktionsgemisch; und
- Hinzufügen von 1-Vinylimidazol zu dem Reaktionsgemisch;
- Reagieren der Verbindung gemäß Formel (IV) mit 2-(Chlormethyl)oxiran und Trimethylamin (Et₃N), um die Verbindung gemäß Formel (V) auszubilden; 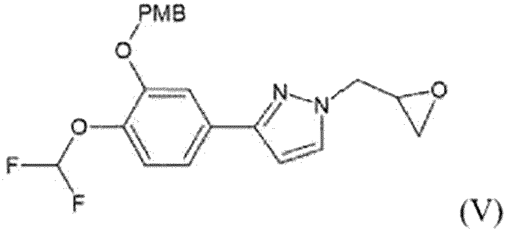
- Reagieren der Verbindung gemäß Formel (V) mit Morpholin, um die Verbindung gemäß Formel (VI) auszubilden; und 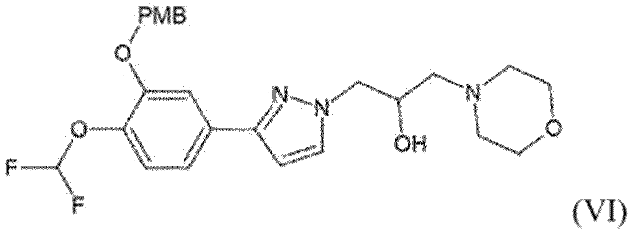
- Reagieren der Verbindung gemäß Formel (VI) mit Trifluoressigsäure (TFA), um die Verbindung gemäß Formel (I') auszubilden.

12. Verfahren nach Anspruch 11, wobei der Schritt des Hinzufügens von Toluol-4-sulfonsäurehydrazid zu der gelösten Verbindung gemäß Formel (III), wobei ein Reaktionsgemisch ausgebildet wird, bei einer Temperatur in dem Bereich von 15 °C bis 40 °C, vorzugsweise bei einer Temperatur in dem Bereich von 15 °C bis 30 °C, mehr bevorzugt bei einer Temperatur von etwa 20 °C, durchgeführt wird.

13. Verfahren nach Anspruch 11 oder 12, wobei der Schritt des Hinzufügens von Toluol-4-sulfonsäurehydrazid zu der gelösten Verbindung gemäß Formel (III), wobei ein Reaktionsgemisch ausgebildet wird, für mindestens 45 Minuten, mehr bevorzugt für mindestens 55 Minuten, mehr bevorzugt für etwa 60 Minuten, durchgeführt wird.

14. Verfahren nach einem der Ansprüche 11 bis 13, wobei der Schritt des Hinzufügens von Natriumhydroxid (NaOH) zu dem Reaktionsgemisch für mindestens 10 Minuten, vorzugsweise für mindestens 15 Minuten, mehr bevorzugt für mindestens 20 Minuten, durchgeführt wird.

15. Verfahren nach einem der Ansprüche 11 bis 14, wobei der Schritt des Reagierens der Verbindung gemäß Formel (V) mit Morpholin, um die Verbindung gemäß Formel (VI) auszubilden, bei einer Temperatur in dem Bereich von 30 °C bis 80 °C, vorzugsweise bei einer Temperatur in dem Bereich von 40 °C bis 70 °C, mehr bevorzugt bei einer Temperatur in dem Bereich von 50 °C bis 60 °C, durchgeführt wird.

## Revendications

1. Composé selon la formule (I), ou un stéréo-isomère, tautomère, racémique, sel, hydrate, forme N-oxyde ou solvate de celui-ci, 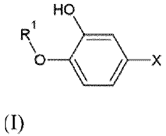 dans lequel :
R¹ = -CH₃ ou -CHF₂ ;
X= 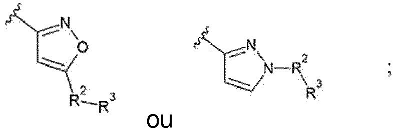
R² = -CO, -C=O(CH₂), -CH(OH)-CH₂, -CH₂-C=O, -CH₂-CH₂-C=O, -CH₂-CH(OH)-CH₂, -CH₂CH(OCOR⁴)-CH₂ ;
R³ = 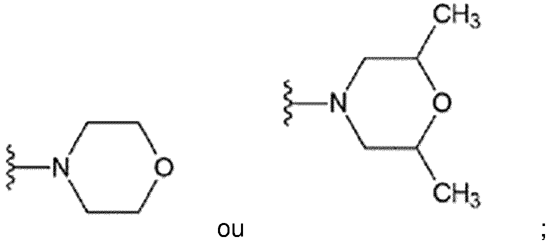 et
R⁴ = alkyle en C₁-C₃,

2. Composé selon la revendication 1, dans lequel R⁴ est -CH₃.

3. Composé selon l'une quelconque des revendications précédentes, dans lequel ledit composé est choisi parmi la liste comprenant

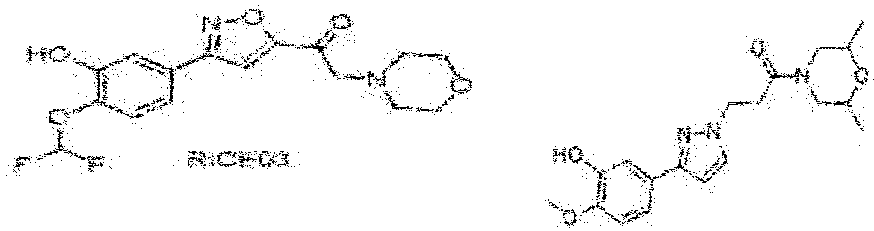

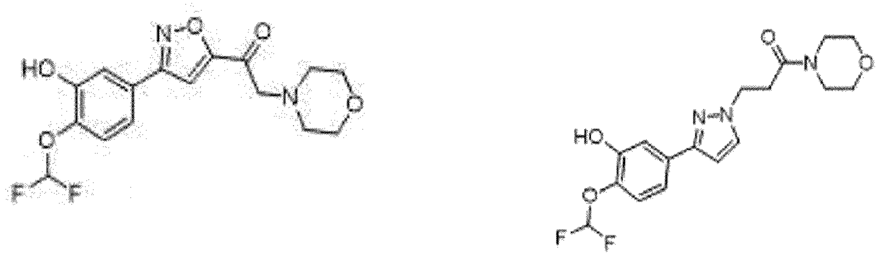

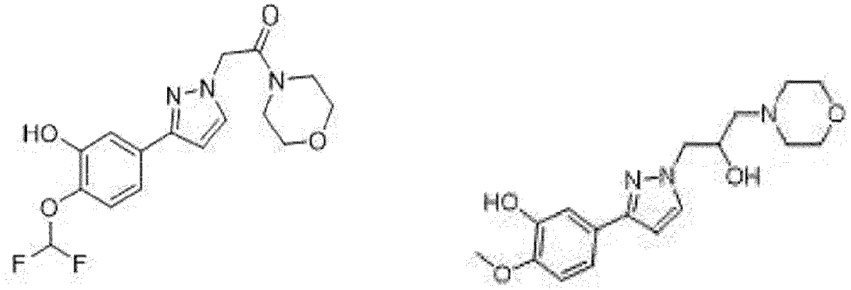

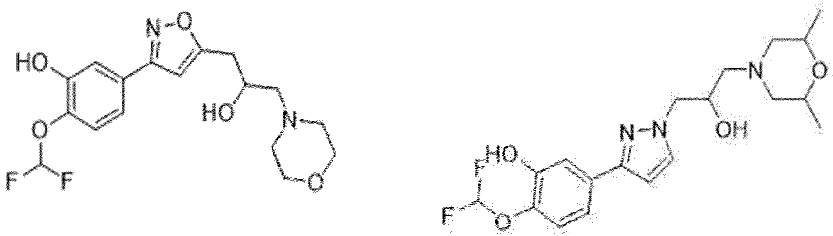

4. Composition pharmaceutique comprenant au moins un composé selon l'une quelconque des revendications 1 à 3 et un support pharmaceutique acceptable.

5. Composé selon l'une quelconque des revendications 1 à 3, ou composition pharmaceutique selon la revendication 4, pour une utilisation en médecine humaine ou vétérinaire.

6. Composé selon l'une quelconque des revendications 1 à 3, ou composition pharmaceutique selon la revendication 4, pour une utilisation en tant que médicament ayant une activité inhibitrice de la PDE4D.

7. Composé selon l'une quelconque des revendications 1 à 3 ou composition pharmaceutique selon la revendication 4, pour utilisation dans la prévention et/ou le traitement et/ou la guérison d'un trouble neurodégénératif chez un sujet.

8. Composé ou composition pharmaceutique pour utilisation selon la revendication 7, dans lequel/laquelle le trouble neurodégénératif est choisi parmi la liste comprenant : la maladie d'Alzheimer (AD), la maladie de Parkinson (PD), la maladie de Huntington, la maladie de Lou Gehrig (ALS), les accidents vasculaires cérébraux, les lésions cérébrales traumatiques, les lésions de la moelle épinière, les troubles cognitifs légers et les maladies démyélinisantes telles que les maladies démyélinisantes périphériques, et la sclérose en plaques (MS).

9. Composé ou composition pharmaceutique pour utilisation selon la revendication 8, dans lequel/laquelle la sclérose en plaques est une sclérose en plaques progressive telle que choisie dans le groupe comprenant la sclérose en plaques primaire progressive, la sclérose en plaques secondaire progressive et la sclérose en plaques récurrente-rémittente.

10. Inhibiteur de la PDE4D comprenant un composé selon l'une quelconque des revendications 1 à 3 ou une composition pharmaceutique selon la revendication 4, dans lequel l'inhibiteur de la PDE4D inhibe sélectivement les isoformes de type D de la PDE4.

11. Procédé de synthèse d'un composé selon l'une quelconque des revendications 1 à 2, comprenant les étapes consistant à :
- fournir un composé selon la formule (II) ; 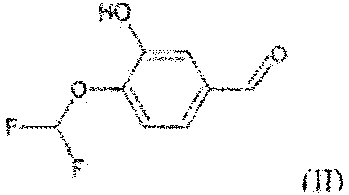
- faire réagir le composé selon la formule (II) avec le chlorure de 4-méthoxybenzyle (PMBC1) formant un composé selon la formule (III) ; 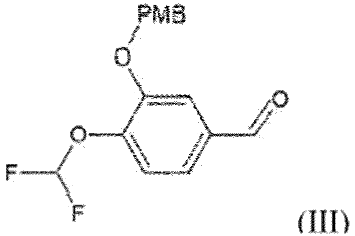
- dissoudre le composé selon la formule (III) dans l'acétonitrile (MeCN) ;
- faire réagir le composé dissous selon la formule (III) pour former le composé selon la formule (IV), 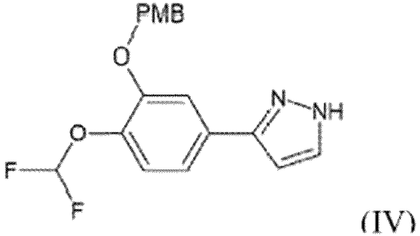 dans lequel l'étape de réaction comprend :
- l'ajout d'hydrazide de l'acide toluène-4-sulfonique au composé dissous selon la formule (III) pour former un mélange réactionnel ;
- l'ajout de l'hydroxyde de sodium (NaOH) au mélange réactionnel ; et
- l'ajout du 1-vinlyimidazole au mélange réactionnel ;
- la réaction du composé selon la formule (IV) avec le 2-(chlorométhyl)oxirane et la triméthylamine (EtsN) pour former le composé selon la formule (V) ; 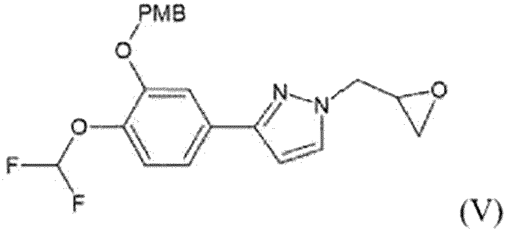
- la réaction du composé selon la formule (V) avec la morpholine pour former le composé selon la formule (VI) ; et 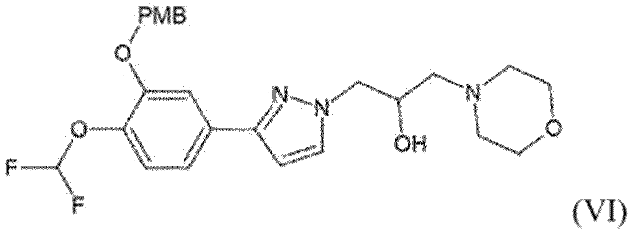
- la réaction du composé selon la formule (VI) avec l'acide trifluoroacétique (TFA) pour former le composé selon la formule (I').

12. Procédé selon la revendication 11, dans lequel l'étape consistant à ajouter l'hydrazide de l'acide toluène-4-sulfonique au composé dissous selon la formule (III) pour former un mélange réactionnel est effectuée à une température dans la plage de 15 °C à 40 °C, de préférence à une température dans la plage de 15 °C à 30 °C, plus préférablement à une température d'environ 20 °C.

13. Procédé selon les revendications 11 ou 12, dans lequel l'étape consistant à ajouter l'hydrazide de l'acide toluène-4-sulfonique au composé dissous selon la formule (III) formant un mélange réactionnel est effectuée pendant au moins 45 minutes, de préférence pendant au moins 55 minutes, plus préférablement pendant environ 60 minutes.

14. Procédé selon l'une quelconque des revendications 11 à 13, dans lequel l'étape consistant à ajouter l'hydroxyde de sodium (NaOH) au mélange réactionnel est effectuée pendant au moins 10 minutes, de préférence pendant au moins 15 minutes, plus préférablement pendant au moins 20 minutes.

15. Procédé selon l'une quelconque des revendications 11 à 14, dans lequel l'étape consistant à faire réagir le composé selon la formule (V) avec la morpholine pour former le composé selon la formule (VI) est effectuée à une température dans la plage de 30 °C à 80 °C, de préférence effectuée à une température dans la plage de 40 °C à 70 °C, plus préférablement effectuée à une température dans la plage de 50 °C à 60 °C.
